# EUROPEAN PATENT SPECIFICATION

(11) **EP 3 580 190 B1**
(45) Date of publication and mention of the grant of the patent: **04.08.2021**
(21) Application number: 18702715.6
(22) Date of filing: 07.02.2018
(51) Int. Cl.: C07B 59/00, C07F 3/12

(54) **IN VIVO STABLE HG-197(M) COMPOUNDS, METHOD FOR THE PRODUCTION THEREOF AND USE THEREOF IN NUCLEAR MEDICAL DIAGNOSTICS AND ENDORADIONUCLIDE THERAPY (THERANOSTICS)**
IN-VIVO STABILE HG-197(M)-VERBINDUNGEN, VERFAHREN ZUR DEREN HERSTELLUNG, UND VERWENDUNG IN DER NUKLEARMEDIZINISCHEN DIAGNOSTIK UND ENDORADIONNUKLIDTHERAPIE
DERIVÉS STABLES DE HG-197(M), LEUR UTILISATION DANS LE CADRE DU DIAGNOSTIC EN MEDECINE NUCLEAIRE ET DE LA THERAPIE A ENDORADIONUCLEIDES, ET PROCEDE POUR PRODUIRE

(30) Priority: 08.02.2017 EP 17155213
(43) Date of publication of application: 18.12.2019
(73) Proprietor: Helmholtz-Zentrum Dresden - Rossendorf e.V., 01328 Dresden (DE)
(72) Inventor: PIETZSCH, Hans-Jürgen, 01809 Heidenau (DE); WALTHER, Martin, 01458 Ottendorf-Okrilla (DE); WÜNSCHE, Thomas, 1183DE Amstelveen (NL)
(74) Representative: Kailuweit & Uhlemann Patentanwälte Partnerschaft mbB
(86) International application number: PCT/EP2018/052996
(87) International publication number: WO 2018/146116

(56) References cited:
- GB-A- 1 220 500
- DATABASE CAPLUS [Online] CHEMICAL ABSTRACTS SERVICE, COLUMBUS, OHIO, US; 1971, XP002779646, retrieved from STN Database accession no. 1971:470067
- DATABASE CAPLUS [Online] CHEMICAL ABSTRACTS SERVICE, COLUMBUS, OHIO, US; 1971, XP002779647, retrieved from STN Database accession no. 1971:120864

## Description

The present invention relates to *in vivo* stable ^{197(m)}Hg compounds according to formulas (I), (Ia), (Ib), (Ic), (Ia_{bridge}), (Ib_{bridge}), (Ic_{bridge}) or (VI)
for use in nuclear medical diagnostics and endoradionuclide therapy (theranostics), particularly the treatment of cancer, a method for the production of the ^{197(m)}Hg compounds comprising the step of radiolabeling of organic precursor compounds with NCA ^{197(m)}Hg by electrophilic substitution; and the use of that precursor compounds in the production of the *in vivo* stable ^{197(m)}Hg compounds.

### State of the art

There has been a continuing need for effective radioisotopes in nuclear medical diagnostics and endoradionuclide therapy (theranostics).

The interest in the mercury isotope ^{197(m)}Hg was awakened primarily by the decay characteristics of both nuclear isomers, like convenient half-life ^{197m}Hg (T_{1/2} = 23.8 h, E_{γ} 134 keV, 34%) and ¹⁹⁷Hg (T_{1/2} = 64.14 h, E_{γ} 77 keV, 19%), low energy gamma radiations useful for diagnosis and numerous Auger and conversion electrons with high potential for cancer therapy.

Mercury (Hg) radioisotopes with low specific activity have been used for imaging from the 1950s (Greif *et al.,* 1956, Sodee 1964) until the late 1960s (Matricali, 1969) exemplary for brain scanning and cancer imaging. Greif *et al.* disclose the use of ¹⁹⁷Hg labelled Neohydrin® as radionuclide in nuclear medical diagnostics of the kidney (Greif *et al.* 1956). The ¹⁹⁷Hg labelled Neohydrin® was produced by n/gamma reaction of enriched ¹⁹⁶Hg in a reactor, wherein a low specific activity of 1 GB/µmol was achieved. Furthermore, the product was contaminated with ²⁰³Hg.

Alternatively, Walther *et al.* proved the feasibility of the production of the no carrier added (NCA) radionuclide ^{197m}Hg from gold at low proton energies in sufficient quantity and quality for imaging and experimental therapeutic purposes (Walther *et al.* 2015). The production of the no carrier added (NCA) radionuclide ^{197m}Hg was carried out through proton induced nuclear reactions on gold via the ¹⁹⁷Au(p,n)^{197(m)}Hg reaction in quantities up to about each 100 MBq, wherein Au superseded the expensive enrichment for the target material. For separation of ^{197m}Hg and ¹⁹⁷Hg from the predominant part of the target material a liquid-liquid extraction method was applied.

Walther *et al.* discloses a resin based method for the separation of Hg radionuclides from Au targets *via* di-(2-ethylhexyl)orthophosphoric acid (HDEHP) on an inert support (Walther *et al.* 2016). Advantageously, the separation method exhibits a higher separation factor, a better handling and the possibility for automation, which significantly improves radiation protection, significantly lower product losses during the separation, and convenient recycling of the gold target material.

The use of radionuclides in nuclear medical diagnostics and endoradionuclide therapy (theranostics) requires the production of *in vivo* stable labeling units. For the clinical chelation therapy of mercury poisoning the sulfur-containing chelating agents meso-dimercaptosuccinic acid (DMSA, Chemet®) and dimercaptopropanesulfonic acid (DMPS, Dimaval®) are generally used (George *et al.* 2004). However, George *et al.* discloses the instability of the formed Hg chelate complexes with DMSA and DMPS.

Thus, there remains a need for *in vivo* stable ^{197(m)}Hg compounds.

Griffith *et al.* discloses the organometallic mercury compound Chlormerodrin ((3-Carbaoylamino-2-methoxypropyl)-chloromercury, Neohydrin®), a mercurial diuretic, which was used in the treatment of chronic congestive heart failure (Griffith *et al.* 1956). Its radiolabeled derivative ²⁰³Hg-Neohydrin has been used for tumor diagnostics (Mishkin 1966). The organometallic mercury compound Merbromin (2',7'-Dibromo-5'-(hydroxymercurio)fluorescein disodium salt, Mercurochrome®) has been used as antiseptic. Because of its mercury content it is no longer sold in Switzerland, France, Germany and the United States.

GB 1 220 500 A discloses a radioactive Di-flouresceinyl-Hg compound.

US 1,672,615 A discloses the antiseptic and antifungal agent Thiomersal (Ethyl(2-mercaptobenzoato-(2-)-O,S) mercurate 1-sodium, Merthiolate®) or thimerosal, respectively, which has been used as a preservative in vaccines, immunoglobulin preparations, skin test antigens, antivenins, ophthalmic and nasal products and tattoo inks. Furthermore, US 1,672,615 A describes a method for the synthesis of water-soluble compounds of alkyl mercuric compounds, which comprises treating a mercuric compound, in which one valence bond is attached to a substituent of other than the sulphur family and the other valence bond is attached to a carbon atom of an alkyl substituent, with an organic compound containing both an acid substituent and a sulfhydryl group directly attached to a carbon atom.

The radiolabeled compound Merisoprol acetate ¹⁹⁷Hg (hydroxy(2-hydroxypropyl)¹⁹⁷mercury, Merprane®) or Merisoprol acetate ²⁰³Hg, respectively, has been used for diagnosis of renal function.

Disadvantages of the disclosed organometallic mercury compounds are the contamination with ²⁰³Hg and the toxicity because of the high Hg content.

### Object of the present invention

The invention has the object of finding organometallic ^{197(m)}Hg compounds with high purity and high specific activity.

### Character of the present invention

The objective of the invention is solved by
a ^{197(m)}Hg compound according to one of the following formulas (I), (Ia), (Ib), (Ic), (Ia_{bridge}), (Ib_{bridge}), (Ic_{bridge}) or (VI) 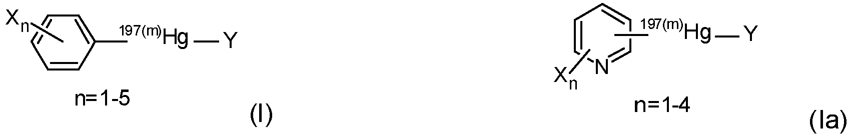 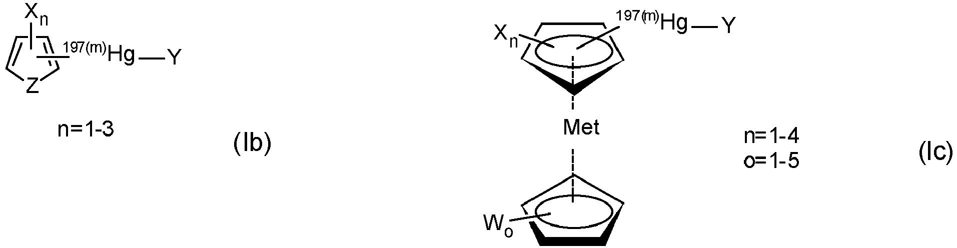 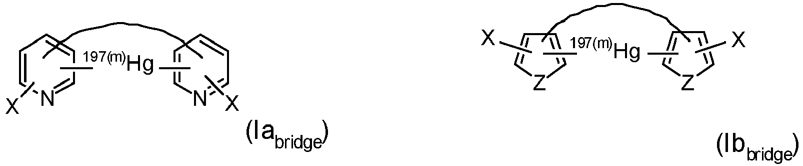 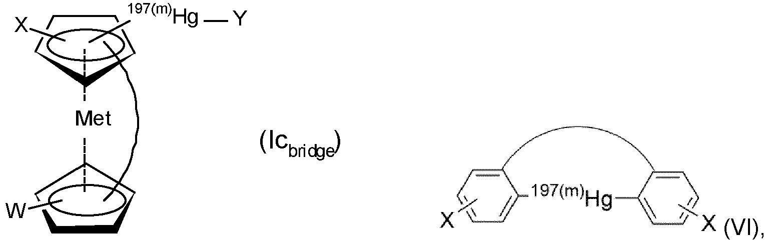
- wherein each X and each W are independently selected from H, unsubstituted or substituted alkyl groups, alkoxy groups with formula -OR¹, amide groups with formula -CON(R¹)₂, carboxy groups with formula -COOR¹, aryl and heteroaryl groups,
- wherein R¹ is selected from H, unsubstituted or substituted C1 to C15-alkyl, succinimidyl, -aryl or -heteroaryl groups,
- wherein Y is selected from substituted dithiocarbamates, substituted thiolates, unsubstituted or substituted phenyl and other aryl or heteroaryl group,
- wherein Z is selected from CH, S, N, and O, and
- wherein Met is selected from Fe, Cr, Mn, Mo, Ru, and Rh.
^{197(m)}Hg according to the invention is a radionuclide comprising at least one of the two radioactive, γ-emitting nuclear isomers ¹⁹⁷Hg in the ground state and ^{197m}Hg in the excited state, wherein m stands for metastable. The nuclear isomer in the excited state, ^{197m}Hg, emits during its nuclear isomeric transition with a half-life (T_{1/2}) of 23.8 h, a low-energy gamma radiation (E_{γ}) of 134 keV with 34% probability and conversion electrons with energies between 82 keV and 150 keV. The radioactive Hg isotope ¹⁹⁷Hg exhibits a half-life (T_{1/2}) of 64.14 h, a low-energy gamma radiation (E_{γ}) of 77.4 keV with 19% probability and emission of Auger- and conversion electrons.

Preferably, the radionuclide ^{197(m)}Hg comprises a molar ratio of ^{197m}Hg to ¹⁹⁷Hg of 1:1 to 2:1.

Advantageously, the contamination of the ^{197(m)}Hg compound according to formula (I) with other radioactive and non-radioactive Hg isotopes is excluded by the production method according to the invention. Preferably the content of other radioactive Hg isotopes (for example ¹⁹⁴Hg, ¹⁹⁵Hg and ²⁰³Hg) is less than 10⁻⁶% of the ^{197(m)}Hg content (w/w). Preferably the content of non-radioactive Hg isotopes (¹⁹⁶Hg, ¹⁹⁸Hg, ¹⁹⁹Hg, ²⁰⁰Hg, ²⁰¹Hg, ²⁰²Hg and ²⁰⁴Hg) is below the detection limit of inductively coupled plasma mass spectrometry (ICP-MS) of 1·10⁻¹² (w/w).

Preferably the ^{197(m)}Hg compound according to the invention is produced by the no carrier added (NCA) method as described below.

As used herein, the term "aryl group" refers to unsubstituted or substituted, aromatic hydrocarbon groups. In an embodiment aryl groups are C1 to C18 groups, preferred 5 to 12 groups. In a further embodiment aryl groups are selected from a phenyl group, a tolyl group, a xylyl group and a naphthyl group.

As used herein, the term "heteroaryl group" refers to unsubstituted or substituted, aromatic hydrocarbon groups with at least one heteroatom. Heteroatoms are selected from nitrogen, oxygen, phosphor and sulfur. In an embodiment heteroaryl groups are C1 to C18 groups, preferred 5 to 12 groups. In a further embodiment heteroaryl groups are selected from a furanyl group, pyrrolyl group, thienyl group, oxazolyl group, thiazolyl group, imidazolyl group, pyrazolyl group, pyrimidyl group, pyridazinyl group and indolyl group. In another embodiment heteroaryl groups are selected from 2-Methylbenzfuranyl, 2-Methylbenzothiazyl and 2-Methylthianaphthenyl.

In some embodiments Y in formulas (I), (Ia), (lb), (Ic) or (Ic_{bridge}) and the other substituent of the ^{197(m)}Hg are identical.

In some other embodiments they are not identical.

The phenyl ring in the ^{197(m)}Hg compound according to formula (I) is substituted with 1 to 5 Xₙ, wherein Xₙ is selected from X₁, X₂, X₃, X₄, X₅. Thus, X₁ to X₅ are independently selected from H, unsubstituted or substituted alkyl, alkoxy (-OR¹), amide (-CON(R¹)₂), carboxy (-COOR¹), aryl and heteroaryl groups.

Unsubstituted alkyl, alkoxy (-OR¹), amide (-CON(R¹)₂), carboxy (-COOR¹), aryl or heteroaryl groups according to the invention are hydrocarbon groups without side chains. As used herein, the term "side chains" refers to atoms or atom groups that are attached to a core part of a molecule or the alkyl, alkoxy (-OR¹), amide (-CON(R¹)₂), carboxy (-COOR¹), aryl or heteroaryl groups, respectively.

Substituted according to the invention is the replacement of at least one hydrogen atom by an atom or group of atoms on a hydrocarbon compound. The atom or group of atoms is preferably selected from C1 to C15-alkyl, -aryl, -heteroaryl, -alkoxy (-OR²), -carbonyl (-COR²), -amino (-N(R²)₂ or -NHR²), nitro (-NO₂), phosphate groups or halogenides, wherein R² is selected from H, unsubstituted or substituted C1 to C15-alkyl, -aryl or -heteroaryl groups. The carbonyl group can be an aldehyde group (-CHO), a keto group (-COR²), a carboxylic acid group (-COOH), carboxylate ester groups (-COOR¹) or an amide (-CON(R²)₂).

In an embodiment Xₙ comprises between 1 and 50 carbon atoms, preferred between 1 and 25 carbon atoms, especially preferred between 1 and 10 carbon atoms.

In some embodiments Xₙ is selected from unsubstituted or substituted alkyl, alkoxy (-OR¹), amide (-CON(R¹)₂), carboxy (-COOR¹), aryl or heteroaryl groups.

In a preferred embodiment Xₙ or X are selected from substituted amide groups.

As used herein, the term "alkyl group" refers to unbranched or branched, unsubstituted or substituted hydrocarbon groups. In an embodiment alkyl groups are C1 to C10 groups, preferred C1 to C3 groups.

In a further embodiment alkyl groups are selected from a methyl group, an ethyl group, a propyl group, an isopropyl group, a pentyl group and a hexyl group.

In a further embodiment Xₙ comprises at least one heteroatom, preferred two heteroatoms. Heteroatoms are selected from nitrogen, oxygen, phosphor and sulfur.

As used herein, the term "alkoxy group" refers to unbranched or branched, unsubstituted or substituted hydrocarbon groups, wherein at least one oxygen is singular bonded to R¹, wherein R¹ is selected from H, unsubstituted or substituted alkyl, -aryl or -heteroaryl groups. In an embodiment alkoxy groups are C1 to C10 groups, preferred 1 to 3 groups.

In a further embodiment alkoxyl groups are selected from a methoxy group, an ethoxy group and a propoxy group.

As used herein, the term "amide group" refers to unbranched or branched, unsubstituted or substituted hydrocarbon groups, wherein at least one amide group is singular bonded to R¹.

As used herein, the term "carboxy group" refers to unbranched or branched, unsubstituted or substituted hydrocarbon groups, wherein at least one carboxy group is singular bonded to R¹.

In a preferred embodiment the ^{197(m)}Hg compound according to formula (I) is substituted with 1 to 3 Xₙ, wherein Xₙ is selected from X₁, X₂ and X₃ as described above. In a mostly preferred embodiment the ^{197(m)}Hg compound according to formula (I) is substituted with one Xₙ or X, respectively, as shown in formula (I') 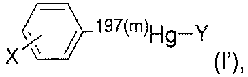
wherein X is selected from H, unsubstituted or substituted alkyl, -alkoxy (-OR¹), -amide (-CON(R¹)₂), -carboxy (-COOR¹), -aryl or -heteroaryl groups,
wherein Y is selected from substituted dithiocarbamates, substituted thiolates, unsubstituted or substituted phenyl and other aryl or heteroaryl groups,
wherein R¹ is selected from H, unsubstituted or substituted C1 to C15-alkyl, succinimidyl, -aryl or -heteroaryl groups.

In a further embodiment the ^{197(m)}Hg compound according to formula (I') is substituted with X in ortho-, meta- or para-position, preferred in para-position as shown in formula (I") 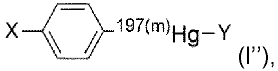
wherein X is selected from H, unsubstituted or substituted alkyl, alkoxy (-OR¹), amide (-CON(R¹)₂), carboxy (-COOR¹), aryl or heteroaryl groups,
wherein Y is selected from substituted dithiocarbamates, substituted thiolates, unsubstituted or substituted phenyl and other aryl or heteroaryl groups,
wherein R¹ is selected from H, unsubstituted or substituted C1 to C15-alkyl, succinimidyl, -aryl or -heteroaryl groups.

In a further embodiment Y comprises between 1 and 50, preferred between 1 and 25 carbon atoms, especially preferred between 1 and 10 carbon atoms.

In a further embodiment Y comprises at least one heteroatom, preferred 1 to 6 heteroatoms. Heteroatoms are selected from nitrogen, oxygen, phosphor and sulfur.

Preferably -Y in formula (I), (I') or (I") is selected from substituted dithiocarbamates according to formula (II) 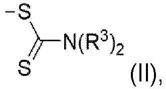
wherein R³ is selected from H, unsubstituted or substituted alkyl, alkoxy (-OR⁴), amide (-CON(R⁴)₂), carboxy (-COOR⁴), aryl or heteroaryl groups,
wherein R⁴ is selected from H, unsubstituted or substituted C1 to C15-alkyl, -aryl or -heteroaryl groups. The two R³ are selected independently.

In a further embodiment R³ is selected from unsubstituted or substituted alkyl, alkoxy (-OR⁴), amide (-CON(R⁴)₂), carboxy (-COOR⁴), aryl or heteroaryl groups.

In a preferred embodiment R³ of the substituted dithiocarbamates is selected from substituted amide (-CON(R⁴)₂) or carboxy (-COOR⁴) groups.

In a further embodiment -Y in formula (I), (I') or (I") is selected from substituted thiolates according to formula (III)

-SR⁵ (III),

wherein R⁵ is selected from H, unsubstituted or substituted alkyl, alkoxy (-OR⁶), amide (-CON(R⁶)₂), carboxy (-COOR⁶), aryl or heteroaryl groups,
wherein R⁶ is selected from H, unsubstituted or substituted C1 to C15-alkyl, -aryl or -heteroaryl groups.

In a further embodiment R⁵ of the substituted thiolates is selected from substituted amide (-CON(R⁶)₂) or carboxy (-COOR⁶) groups.

In a further embodiment of the compound of formula (I) -Y is selected from unsubstituted or substituted phenyl groups according to formula (IV) 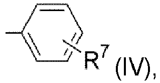 resulting in a compound according to formula (IV') 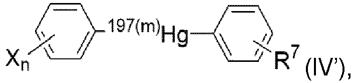
wherein R⁷ is selected from H, unsubstituted or substituted alkyl, alkoxy (-OR⁸), amide (-CON(R⁸)₂), carboxy (-COOR⁸), aryl or heteroaryl groups,
wherein R⁸ is selected from H, unsubstituted or substituted C1 to C15-alkyl, succinimidyl, -aryl or -heteroaryl groups and
wherein X is selected from H, unsubstituted or substituted alkyl, alkoxy (-OR¹), amide (-CON(R¹)₂), carboxy (-COOR¹), aryl or heteroaryl groups,
n is 1 to 5.

In a further embodiment R⁷ of the unsubstituted or substituted phenyl groups is selected from substituted amide (-CON(R⁸)₂) or carboxy (-COOR⁸) groups.

In a further embodiment the ^{197(m)}Hg compound according to formula (IV) is substituted with R⁷ in ortho-, meta- or para-position.

In a further embodiment Y is selected from unsubstituted or substituted phenyl groups according to formula (IV), wherein R⁷ and Xₙ are not identically.

In a further embodiment of the compound of formula (I) Y is selected from unsubstituted or substituted phenyl groups according to formula (IV), wherein n is 1 and wherein R⁷ and X are identically resulting in a compound according to formula (V) 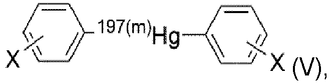
wherein X is selected from H, unsubstituted or substituted alkyl, alkoxy (-OR¹), amide (-CON(R¹)₂), carboxy (-COOR¹), aryl or heteroaryl groups,
wherein R¹ is selected from H, unsubstituted or substituted C1 to C15-alkyl, succinimidyl, -aryl or -heteroaryl groups.

In further preferred embodiments the compounds according the invention are selected from compounds according to formula (Ia'), (Ib') and (Ic'): 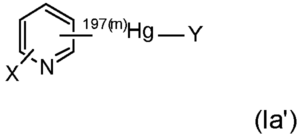 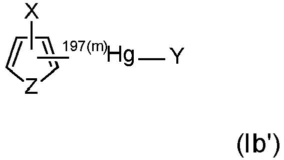 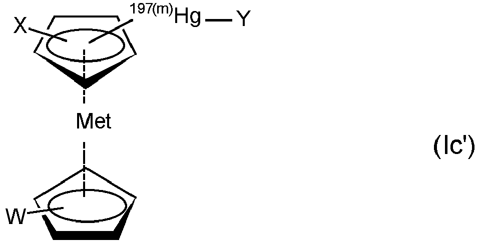
wherein each X and W are independently selected from H, unsubstituted or substituted alkyl groups, alkoxy groups with formula -OR¹, amide groups with formula -CON(R¹)₂, carboxy groups with formula -COOR¹, aryl or heteroaryl groups,
wherein Y is selected from substituted dithiocarbamates, substituted thiolates, unsubstituted or substituted phenyl and other aryl or heteroaryl groups,
wherein R¹ is selected from H, unsubstituted or substituted C1 to C15-alkyl, succinimidyl, -aryl or -heteroaryl groups,
wherein Z is selected from CH, S, N, and O,
wherein Met is selected from Fe, Cr, Mn, Mo, Ru and Rh

In some embodiments the resulting ^{197(m)}Hg-compounds have one of the following formulas:

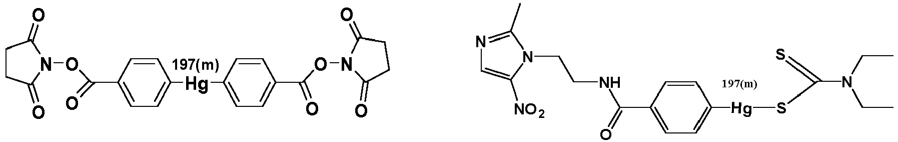

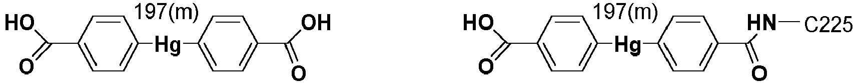

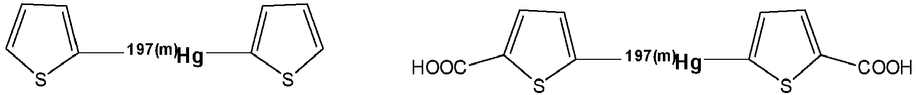

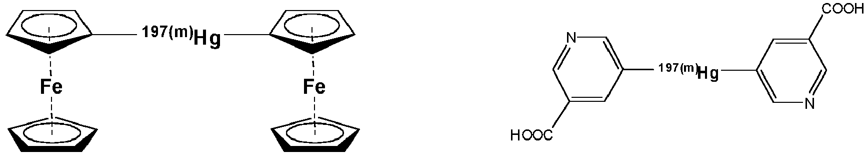

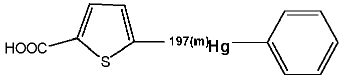

In the compounds according to formulas (Ia_{bridge}), (Ib_{bridge}), (Ic_{bridge}) or (VI) some ^{197(m)}Hg-substituents are linked by at least one aliphatic or aromatic spacer molecule as shown in formulas (Ia_{bridge}), (Ib_{bridge}) (Ic_{bridge}), or (VI).

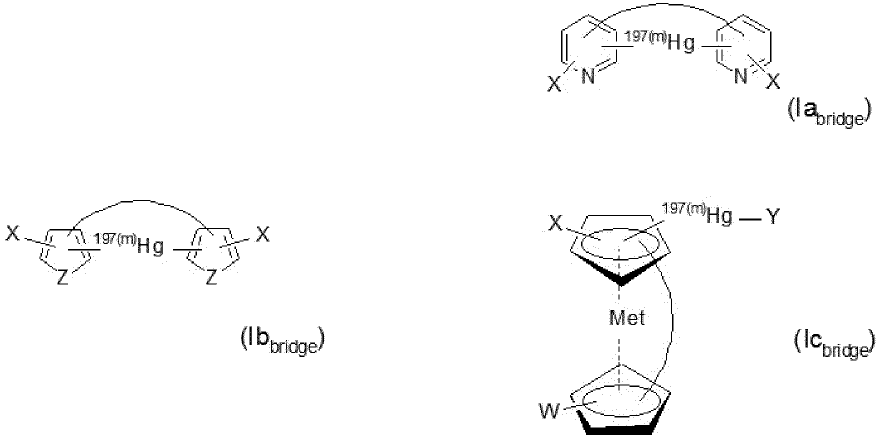

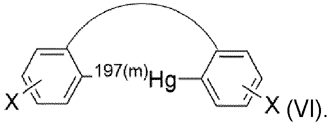

In a preferred embodiment the aliphatic or aromatic spacer molecule is located in ortho-position or meta-position relating to the position of the ^{197(m)}Hg-moiety, at the aryl or heteroaryl groups of formulas (Ia_{bridge}), (Ib_{bridge}) or (Ic_{bridge}).

The aliphatic or aromatic spacer molecule according to the invention is an unsubstituted or substituted C6 to C30-alkyl, -alkoxy (-OR⁹), -amide (-CON(R⁹)₂), -carboxy (-COOR⁹), -aryl or heteroaryl spacer molecule, preferably a C6-alkyl group or a substituted phenyl group.

In an embodiment the ^{197(m)}Hg compounds according to the invention further comprise at least one amino acid, peptide, protein, antibody, oligonucleotide, alkaloid residue and/or aliphatic spacer.

In a further embodiment Xₙ and/or Y further comprise at least one amino acid, peptide, protein, antibody, oligonucleotide, alkaloid residue and/or aliphatic spacer. In an embodiment the aliphatic spacer is selected from polyethylene glycol.

In a further embodiment Xₙ and/or Y comprise at least one amino acid, peptide, protein, antibody, oligonucleotide, alkaloid residue or aliphatic spacer, preferably Xₙ and/or Y comprise 1 to 3 amino acids, peptides, proteins, antibodies, oligonucleotides, alkaloid residues or aliphatic spacers. In a preferred embodiment Xₙ or Y comprises one amino acid, peptide, protein, antibody, oligonucleotide, alkaloid residue or aliphatic spacer.

In an embodiment Xₙ and/or Y comprises one aliphatic spacer and one amino acid, peptide, protein, antibody, oligonucleotide or alkaloid residue.

Advantageously, the ^{197(m)}Hg compounds according to the invention exhibit high purity and high specific activity.

As used herein, the term "purity" refers to the amount of ^{197(m)}Hg compounds according to the invention based on the amount of substance.

As used herein, the term "specific activity" refers to the amount of radioactive decay per time interval (1 decay per second = 1 Becquerel (Bq)) based on the molar amount of substance. The specific activity of the ^{197(m)}Hg compound according to the invention is based on the molar amount of the ^{197(m)}Hg compound. The specific activity can be determined for example by inductively coupled plasma mass spectrometry (ICP-MS).

In an embodiment the ^{197(m)}Hg compounds according to the invention have a specific activity of at least 100 GBq/µmol based on the molar amount of the ^{197(m)}Hg compound, preferred 100 to 1.000 GBq/µmol based on the molar amount of the ^{197(m)}Hg compound.

In an embodiment the ^{197(m)}Hg compounds of the invention can be used in nuclear medical diagnostics and endoradionuclide therapy (theranostic).

In a further embodiment the ^{197(m)}Hg compounds of the invention can be used in the treatment of cancer.

In a further embodiment the ^{197(m)}Hg compounds of the invention can be used for the manufacture of a medicament for endoradionuclide therapy.

In a further embodiment the ^{197(m)}Hg compounds of the invention can be used for the manufacture of a medicament for the treatment of cancer.

In a further embodiment the ^{197(m)}Hg compounds of the invention can be used as an active ingredient for the preparation of a pharmaceutical composition.

The present invention further comprises a pharmaceutical composition comprising a ^{197(m)}Hg compound of the invention.

The present invention further comprises a method for the production of the ^{197(m)}Hg compounds according to the invention comprising the steps:
a) Provision of an organic precursor compound,
b) Synthesis of no carrier added (NCA) ^{197(m)}Hg,
c) Radiolabeling of the organic precursor compound with the no carrier added (NCA) ^{197(m)}Hg by electrophilic substitution.

Advantageously, the method for the production of ^{197(m)}Hg compounds according to the invention is fast and is carried out under moderate conditions. As used herein, the term "fast" refers to periods of a few minutes to a few hours, preferred 5 min to 2 h. As used herein, the term "moderate conditions" refers to moderate temperatures of 25 to 70°C. Advantageously, compounds with the radioactive Hg isotopes ¹⁹⁷Hg and ^{197m}Hg can be synthesized by the method according to the invention and administered to patients for the use in nuclear medical diagnostics and endoradionuclide therapy (theranostic), preferred in the treatment of cancer, before the half-life (T_{1/2} (¹⁹⁷Hg) = 64.14 h, T_{1/2} (^{197m}Hg) = 23.8 h) of the radioactive Hg isotopes has passed. Furthermore advantageously, temperature-sensitive molecules, for example peptides, proteins, nucleic acids or antibodies, are preserved under the moderate conditions.

In an embodiment the method for the production of ^{197(m)}Hg compounds according to the invention is carried out in the order of the steps a), b) and c).

In a further embodiment the method for the production of ^{197(m)}Hg compounds according to the invention is carried out in the order of the steps b), a) and c).

As used herein, the term "organic precursor compound" refers to a hydrocarbon compound comprising at least one heteroatom.

In an embodiment the organic precursor compound is an organotin precursor compound, a boron precursor compound or a silicon precursor compound according to formulas (I_{prec}), (Ia_{prec}), (Ib _{prec}) or (Ic_{prec}) 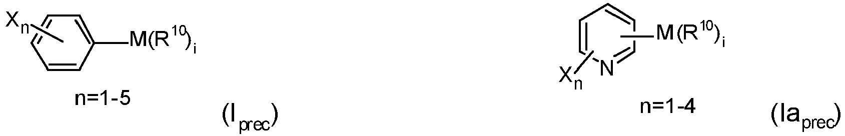 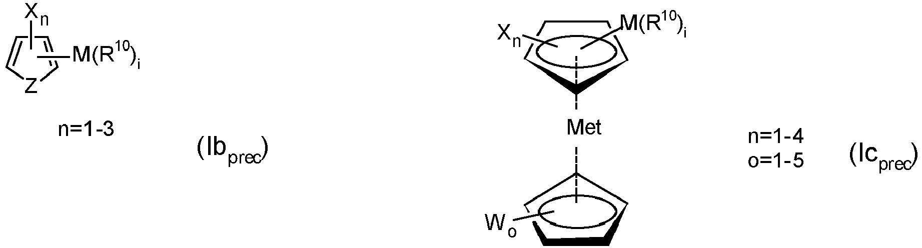
wherein each X and each W are independently selected from H, unsubstituted or substituted alkyl, alkoxy (-OR¹), amide (-CON(R¹)₂), carboxy (-COOR¹), aryl or heteroaryl groups,
wherein R¹ is selected from H, unsubstituted or substituted C1 to C15-alkyl, -aryl or -heteroaryl groups,
Z is selected from CH, S, N, and O,
M is Sn, B or Si;
wherein Met is selected from Fe, Cr, Mn, Mo, Ru and Rh,
R¹⁰ is selected from H, unsubstituted or substituted C1 to C15-alkyl, -aryl or -heteroaryl groups, preferred C1 to C5-alkyl groups;
i is 2 or 3.
Object of the invention is also the use of these organic precursor compounds in the production of the ^{197(m)}Hg compounds according to the invention.

In a preferred embodiment the organic precursor compound is an organotin precursor compound, a boron precursor compound or a silicon precursor compound, wherein n and o are 1, according to formulas (I_{prec}'), (Ia_{prec}'), (Ib_{prec}'), or (Ic_{prec}') 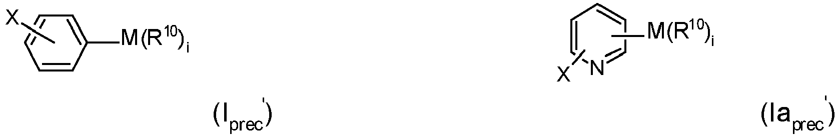 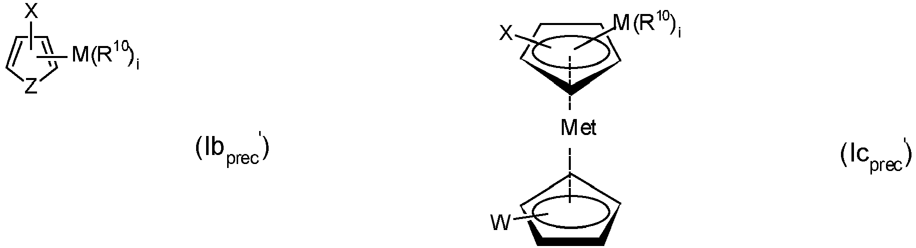
wherein each X and each W are independently selected from H, unsubstituted or substituted alkyl, alkoxy (-OR¹), amide (-CON(R¹)₂), carboxy (-COOR¹), aryl or heteroaryl groups,
wherein R¹ is selected from H, unsubstituted or substituted C1 to C15-alkyl, succinimidyl, -aryl or -heteroaryl groups,
Z is selected from CH, S, N, and O,
M is Sn, B or Si,
wherein Met is selected from Fe, Cr, Mn, Mo, Ru and Rh,
R¹⁰ is selected from H, unsubstituted or substituted C1 to C15-alkyl, -aryl or -heteroaryl groups, preferred C1 to C5-alkyl groups;
i is 2 or 3.

In an embodiment the organic precursor compound according to formulas (I_{prec}'), (Ia_{prec}'), (Ib_{prec}'), or (Ic_{prec}') is substituted with X in ortho-, meta- or para-position, compared to substituent M(R¹⁰)ᵢ.

In a preferred embodiment the organic precursor compound is a tin precursor compound, especially preferred a trialkyl-tin precursor compound. Trialkyl-tin precursor compounds are selected from tri-n-butyl-tin precursor compounds or trimethyl-tin precursor compounds (according to the following formulas):

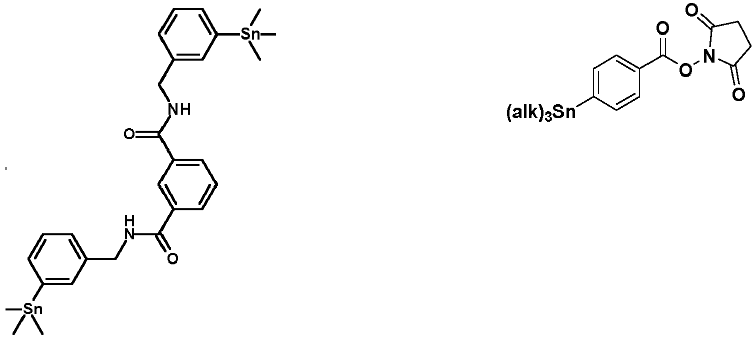

In a further embodiment the organic precursor compound is synthesized by catalytic reaction of the halogen compound. In a preferred embodiment the organic precursor compound is synthesized by catalytic reaction of the halogen compound with an alkyl-tin compound, an alkyl-boron compound or an alkyl-silicon compound.

In a further embodiment the synthesis of NCA ^{197(m)}Hg according to step b) is carried out by irradiation of gold (Au) with a cyclotron. As used herein, the term "no carrier added (NCA)" refers to preparation of a radioactive isotope without the addition of stable isotopes of the element in question.

In a further embodiment the NCA ^{197(m)}Hg synthesised according to step b) is NCA ^{197(m)}HgCl₂.

In a further embodiment the synthesis of NCA ^{197(m)}Hg according to step b) is followed by purification of the NCA ^{197(m)}Hg by liquid-liquid extraction or solid-phase extraction.

In a further embodiment the radiolabeling of the organic precursor compound according to step c) is carried out by addition of NCA ^{197(m)}HgCl₂ to the organic precursor compound.

In a further embodiment the radiolabeling of the organic precursor compound according to step c) is carried out by addition of the NCA ^{197(m)}Hg to the organic precursor compound in a molar ratio of 1:10 to 1:1.000 (n/n).

In a further embodiment the radiolabeling of the organic precursor compound according to step c) is carried out at a pH value between pH 1.0 and 7.0.

In a further embodiment the radiolabeling of the organic precursor compound according to step c) is carried out at a pH value between pH 6.0 and 7.0 to form symmetric ^{197(m)}Hg compounds.

As used herein, the term "symmetric ^{197(m)}Hg compounds" refers to ^{197(m)}Hg compounds, wherein ^{197(m)}Hg exhibits two identical binding partners. Symmetric ^{197(m)}Hg compounds are ^{197(m)}Hg compounds according to formula (V) and (VI).

In a further embodiment the radiolabeling of the organic precursor compound according to step c) is carried out at a pH value between pH 1.0 and 5.0 to form asymmetric ^{197(m)}Hg compounds.

As used herein, the term "asymmetric ^{197(m)}Hg compounds" refers to ^{197(m)}Hg compounds, wherein ^{197(m)}Hg exhibits two different binding partners. Asymmetric ^{197(m)}Hg compounds are ^{197(m)}Hg compounds of the invention, except ^{197(m)}Hg compounds according to formula (V) and (VI).

In a further embodiment the formed asymmetric ^{197(m)}Hg compounds are added to dithiocarbamate ligands to form ^{197(m)}Hg compounds according to formula (II).

In a further embodiment the radiolabeling of the organic precursor compound according to step c) is carried out by addition of dimethyl sulfoxide (DMSO). Advantageously, DMSO increases the solubility of the organic precursor compound.

In a further embodiment the radiolabeling of the organic precursor compound according to step c) is followed by reaction of activated ester groups by ester hydrolysis, reaction with amino groups or reaction with hydroxyl groups of an amino acid, peptide, protein, antibody, oligonucleotide, alkaloid residue and/or aliphatic spacer.

As used herein, the term "activated ester groups" refers to *N*-hydroxysuccinimide (NHS) or tetrafluorophenyl (TFP) ester groups.

In a further embodiment ester hydrolysis is carried out with sodium hydroxide solution.

In a further embodiment reaction of activated ester groups with amino groups of an amino acid, peptide, protein, antibody, oligonucleotide, alkaloid residue and/or aliphatic spacer is carried out at a pH value between pH 8.0 and 9.0.

The present invention further comprises an organic precursor compound according to formulas (I_{prec}), (Ia_{prec}), (Ib_{prec}) or (Ic_{prec}) for the use in the method according to the invention.

The present invention further comprises the ^{197(m)}Hg compounds according to the invention for use in nuclear medical diagnostics and endoradionuclide therapy (theranostics) or for use in the treatment of cancer.

Nuclear medical diagnostics and endoradionuclide therapy (theranostics) includes administering to a subject in need thereof, a pharmaceutical composition containing a therapeutically effective amount of ^{197(m)}Hg compounds according to the invention.

In an embodiment of the compounds for use in the treatment this further comprises a nuclear medical diagnostic of the therapeutic efficacy of the ^{197(m)}Hg compounds according to the invention.

A pharmaceutical composition containing the ^{197(m)}Hg compounds according to the invention typically contains a pharmaceutically acceptable carrier, such as saline. The dose of the ^{197(m)}Hg compounds according to the invention is preferably 1 GBq to 5 GBq. The subject may be a mammal, such as a human.

The dose of 1 GBq to 5 GBq of the ^{197(m)}Hg compounds according to the invention with preferably a specific activity of at least 100 GBq/µmol based on the amount of mercury refers to a dose of 10 nmol to 50 nmol of mercury or 2 µg to 10 µg of mercury, respectively. Mostly preferred the ^{197(m)}Hg compounds according to the invention has a maximal specific activity of 1,000 GBq/µmol, which refers to a dose of 1 nmol to 5 nmol of mercury or 0.2 µg to 1 µg of mercury, respectively. Advantageously, these doses of mercury are in the same order of magnitude as the estimated daily Hg intake of the European and North American general population or clearly below and therefore do not lead to toxic concentrations in patients (Clarkson and Magos 2006).

Although the invention describes various dosages, it will be understood by one skilled in the art that the specific dose level and frequency of dosage for any particular subject in need of treatment may be varied and will depend upon a variety of factors. These factors include the metabolic stability of the ^{197(m)}Hg compounds according to the invention and length of action of that compound, the age, body weight, general health, sex, diet, mode and time of administration, rate of excretion, drug combination, the severity of the particular condition, and the host undergoing therapy. Generally, however, dosage will approximate that which is typical for known methods of administration of the specific compound. Thus, a typical dosage of the ^{197(m)}Hg compounds according to the invention will be about 5 to 50 MBq/kg.

The pharmaceutical compositions and formulations containing the ^{197(m)}Hg compounds according to the invention can be administered systemically. As used herein, "systemic administration" or "administered systemically" refers to compositions or formulations that are introduced into the blood stream of a subject, and travel throughout the body of the subject to reach the part of the subject's body in need of treatment at an effective dose before being degraded by metabolism and excreted. Systemic administration of compositions or formulations can be achieved by intravenously injection.

Pharmaceutical compositions containing the ^{197(m)}Hg compounds according to the invention are prepared for administration and/or storage by mixing the ^{197(m)}Hg compounds according to the invention, after achieving the desired degree of purity, with pharmaceutically and/or physiologically acceptable carriers, auxiliary substances or stabilizers (Remington's Pharmaceutical Sciences) in the form of a lyophilisate or aqueous solutions. The term "pharmaceutically acceptable" or "physiologically acceptable," when used in reference to a carrier, is meant that the carrier, diluent or excipient must be compatible with the other ingredients of the formulation and not deleterious to the recipient thereof.

In general, the pharmaceutical compositions are prepared by uniformly and intimately bringing the active ingredient into association with a liquid carrier or a finely divided solid carrier or both, and then, if necessary, shaping the product into the desired formulation. Acceptable carriers, auxiliary substances or stabilizers are not toxic for the recipient at the dosages and concentrations employed; they include buffers such as phosphate, citrate, tris or sodium acetate and other organic acids; antioxidants such as ascorbic acid; low molecular weight polypeptides (less than approximately 10 residues), proteins such as serum albumin, gelatin or immunoglobulins; hydrophilic polymers such as polyvinylpyrrolidone; amino acids such as glycine, glutamine, asparagine, arginine, leucine or lysine; monosaccharides, disaccharides and other carbohydrates, for example glucose, sucrose, mannose, lactose, citrate, trehalose, maltodextrin or dextrin; chelating agents such as EDTA; sugar alcohols such as mannitol or sorbitol; salt-forming counter-ions such as sodium, and/or non-ionic surface-active substances such as Tween, Pluronics or polyethylene glycol (PEG).

Such pharmaceutical compositions may further contain one or more diluents, fillers, binders, and other excipients, depending on the administration mode and dosage form contemplated.

Examples of therapeutically inert inorganic or organic carriers known to those skilled in the art include, but are not limited to, lactose, corn starch or derivatives thereof, talc, vegetable oils, waxes, fats, polyols such as polyethylene glycol, water, saccharose, alcohols, glycerin and the like. Various preservatives, emulsifiers, dispersants, flavorants, wetting agents, antioxidants, sweeteners, colorants, stabilizers, salts, buffers and the like can also be added, as required to assist in the stabilization of the formulation or to assist in increasing bioavailability of the active ingredient(s). The ^{197(m)}Hg compounds according to the invention can be administered alone, or in various combinations, and in combination with other therapeutic agents. The ^{197(m)}Hg compounds used in the invention are normally stored in solution.

In a further embodiment the recently described embodiments can be combined.

### Figures and Examples

The present invention will now be further explained by the following non-limiting figures and examples.
Fig. 1 shows the fractionated elution of ^{197(m)}Hg mercury chloride in 6 M HCI and ¹⁹⁸Au+¹⁹⁶Au containing chloroauric acid in 0.1 M HCI.
Fig. 2 shows (A) Radiochromatogram of Phenyl-^{197(m)}Hg-dithiocarbamate and (B) UV-chromatogram of non-radioactive Phenyl-Hg-dithiocarbamate (reference).

### General synthetic techniques

All Chemicals were used without further purification and in the highest degree of purity.

Sodium hydroxide in suprapur quality was purchased from Merck (Darmstadt, Germany). Methyl isobutyl ketone (MIBK) was purchased from Sigma-Aldrich (St. Louis, USA). The routine activity measurement was performed with an Isomed 2000 from MED (Nuklear-Medizintechnik Dresden GmbH, Dresden, Germany) calibrated by γ-ray spectroscopy measurements after decaying ^{197m}Hg. ICP-MS measurements were carried out on an ELAN 9000 (PerkinElmer SCIEX, Waltham, USA).

### Gamma-ray spectroscopy

For γ-ray spectroscopy measurements a reverse electrode HPGe detector (CANBERRA GR2018, 19.6% rel. efficiency) in a low-background Pb shielding was used with the sample at 10 cm distance from the detector end cap. It was operated with the software InterWinner version 7.1. The system was calibrated using a mixed standard solution (57Co, 85Sr, 88Y, 60Co, 109Cd, 113Sn, 137Cs, 139Ce, 203Hg, 241Am) with a volume of 0.38 mL in the tip of a 1.5 mL Eppendorf vial. The energy depending detector efficiency was calculated from these calibration points using the algorithms of the spectroscopy software. The samples were measured in similar geometry, but smaller volume of 1 - 10 µl in the tip of a 1.5 mL Eppendorf vial thus, no further corrections were necessary with except of decay correction. Pile-up effects were observed, especially at higher activities. Nevertheless, no corrections are made, because the effects are less than the simple standard deviation and thus negligible. For the determination of Hg-activities only the γ-ray lines > 100 keV have been used, in particular for the isomer 197mHg only the lines ∼ 134 keV and ∼ 165 keV of the isomeric transition and for the isomer 197Hg only the lines ∼ 191 keV and ∼ 269 keV are discussed in the activity calculation.

### NMR and IR spectroscopy

¹H and ¹³C NMR spectra were recorded with a Varian Inova-400 spectrometer. The chemical shifts were reported relative to the standard tetramethylsilane (TMS). IR spectra were measured with a Fisher Scientific Nicolet iS5 FTIR spectrometer.

### Thin layer chromatography (TLC)

Thin layer chromatography was performed using RP18 plates (Merck), developed in a 1:1 mixture H₂O with 0.1% trifluoroacetic acid (TFA) (A) and CH₃CN with 0.1% TFA (B) and analyzed with a Raytest Linearanalyser RITA.

Radio-TLC is the detection of radioactive species separated by TLC with radiation detector to determine the radiochemical purity or to quantify the radioactive species.

The radiochemical yield is the yield of the radionuclide and was calculated by the specific activity of the ^{197(m)}Hg compound divided by the specific activity of the no carrier added (NCA) ^{197(m)}Hg.

### High-performance liquid chromatography (HPLC) measurements

Radiochemical purity was determined by radio-HPLC. All HPLC runs are performed under the same conditions with the same HPLC-equipment. Column: Zorbax C18 column with inner diameter of 8 mm. Mobile phase: H₂O with 0.1% TFA (A) and CH₃CN with 0.1% TFA (B). Flow rate: 3 mL/min. HPLC gradient of B phase: in 0 to 20 min from 45% to 80%, in 20 to 25 minfrom 80% to 100%.

### Mass spectrometry (electrospray ionization (ESI)-MS, Matrix-assisted laser desorption/ ionization (MALDI)-MS)

For mass spectrometry a QuadroLC by Micromass with electrospray ionisation (ESI) mode and a Bruker MALDI-TOF MS instrument (MALDI) were used.

### 1. Synthesis of an organic precursor compound

### N¹,N³-bis(3-iodobenzyl)isophthalamide

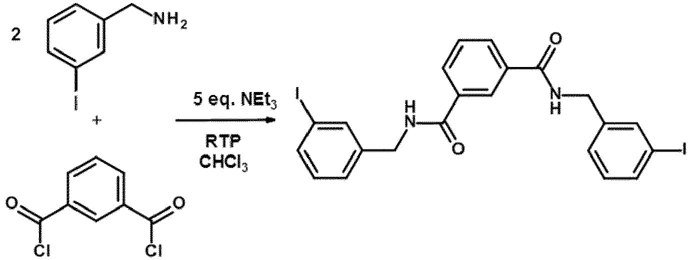

3-iodobenzylamine hydrochloride salt (4 g, 14.84 mmol) was dissolved in chloroform (100 ml) in a 250 ml round-bottomed flask. To this was added triethylamine (10.3 ml, 0.074 mol) followed by isophthaloyl chloride (1.51 g, 7.42 mmol). The flask was fitted with a CaCl₂ drying tube and the colourless solution was left to stir at room temperature overnight. The reaction was monitored by TLC using 19:1 dichloromethane (DCM)/methanol (MeOH). The reaction mixture was washed with 3:1 water/saturated NaHCO_{3(aq.)} (3 x 50 ml), then with 0.1 M HCl_{(aq.)} (3 x 50 ml), then with deionized water (2 x 30 ml). The product is mostly insoluble in chloroform and precipitates during the aqueous washes, thus further dilution with chloroform helps separation. The product was purified by simple recrystallization of cooling the chloroform. Impurities dissolved in the solvent were decanted. This process was repeated to increase yield. The product was washed lightly with cold chloroform and after drying left a white powder (1.02 g, 92% yield).
**¹H** NMR (400 MHz, CDCl₃) *δ* (ppm): 8.23 (s, 1H), 7.92 (dd, *J* = 7.8, 1.6 Hz, 2H), 7.64 (s, 2H), 7.59 (d, *J =* 7.9 Hz, 2H), 7.48 (t, *J =* 7.8 Hz, 1H), 7.28 (s, 1H), 7.04 (d, *J =* 7.8 Hz, 2H), 6.84 (d, *J =* 5.3 Hz, 2H), 4.52 (d, *J =* 5.8 Hz, 4H),
**¹³C** NMR (101 MHz, CDCl₃) *δ* (ppm): 166.57, 140.37, 136.93, 134.52, 130.64, 130.40, 129.27, 127.32, 125.67, 94.78, 43.61.

### N¹,N³-bis(3-(trimethylstannyl)benzyl)isophthalamide

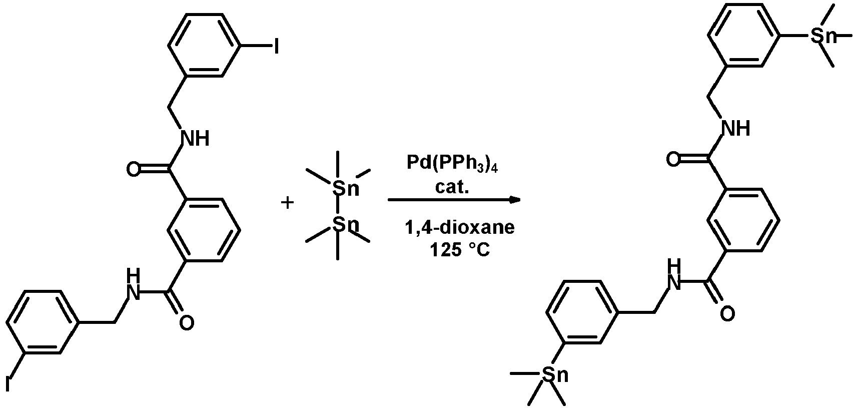

N¹,N³-bis(3-iodobenzyl)isophthalamide (0.97 g, 1.63 mmol) was dissolved in 1,4-dioxane (20 ml) in a 50 ml 3-necked round-bottomed flask. A glass bubbler allowed argon to bubble through the solution with a coiled water condenser attached to the top along with a bubble counter to monitor argon flow. A catalytic amount of tetrakis(triphenylphosphine)palladium(0) (20.4 mg, 16.3 µmol) orange crystals were added forming a clear pale yellow solution. This was followed by an excess of hexamethylditin (3.16 ml, 15.26 mmol). Rinsing of sample phials and addition funnel brought the total solvent volume to 30 ml. The reaction mixture was heated by an oil bath (125 °C) and stirred for 8 h. The reaction was monitored by TLC using 1:1 ethanol (EtOH)/n-hexane. The reaction mixture turned a dark orange with a cloudy precipitate. This was filtered to remove most of the brown precipitate. The solvent was removed by evaporation and the product purified by flash column chromatography using EtOH/n-hexane. Drying yielded a white powder (0.164 g, 15% yield).
**¹H NMR** (400 MHz, d₆-DMSO) *δ* (ppm): 9.11 (broad t, 2H), 8.38 (s, 1H), 8.01 (dd, *J* = 7.8, 1.6 Hz, 2H), 7.58 (t, *J* = 7.8 Hz, 1H), 7.53 - 7.22 (m, 8H), 4.47 (d, *J* = 5.9 Hz, 4H), 0.25 (s, 18H),
**¹³C NMR** (101 MHz, CDCl₃) *δ* (ppm): 166.41, 143.39, 137.31, 135.72, 135.46, 134.92, 130.12, 129.18, 128.61, 128.22, 125.51, 44.68, -9.35.

### 2. Production of no-carrier-added ^{197(m)}Hg

The irradiations were performed at a Cyclone 18/9 cyclotron (IBA, Louvain la Neuve, Belgium, 18 MeV protons) located at Dresden-Rossendorf. A 1.0 mm aluminum foil (high purity aluminum, 99.999%) from Goodfellow (Huntingdon, England) was used as vacuum window. As target material massive high purity gold disks (23 mm diameter, 2 mm thickness, N5 purity 99.999%) were purchased from ESPI (Ashland, USA). Alternative gold targets consisted of a gold foil (12.5 x 12.5 mm, 0.25 mm thickness, 99.99+%) or a small gold disk (10 mm diameter, 0.125 mm thickness, 99.99+%, Pt content: 45± 5 ppm quantified per ICP-MS) between an aluminum disk (22 mm diameter, 1 mm thickness, 99.0%, hard) and an aluminum lid (23 mm diameter, 99.0%, hard) purchased from Goodfellow (Huntingdon, England). Hydrochloric acid (30%) and nitric acid (65%) were purchased from Roth (Karlsruhe, Germany) in Rotipuran® Ultra quality. Deionized water with >18 MΩcm resistivity was prepared by a Milli-Q® system (Millipore, Molsheim, France). LN resin was purchased from Triskem International (Bruz, France). The gold target was irradiated for 120 min with a 25 µA current of 10 MeV protons resulting in 200 MBq of ^{197(m)}Hg. The irradiated gold foil was dissolved in 700 µl of aqua regia (freshly prepared 1 h before EOB from 525 µl 30% HCI + 175 µl 65% HNO₃) at room temperature. The gold disk was completely dissolved after 50 to 60 min. The column preparation was carried out directly before use by loading 3.6 g LN resin slurried with 10 ml of 6 M HCI onto the column and rinsing with additional 30 ml of 6 M HCI. After dilution of the 700 µl product solution with 300 µl 6 M HCI, this mixture was loaded onto the column and eluted with 6 M HCl in 1 ml aliquots.

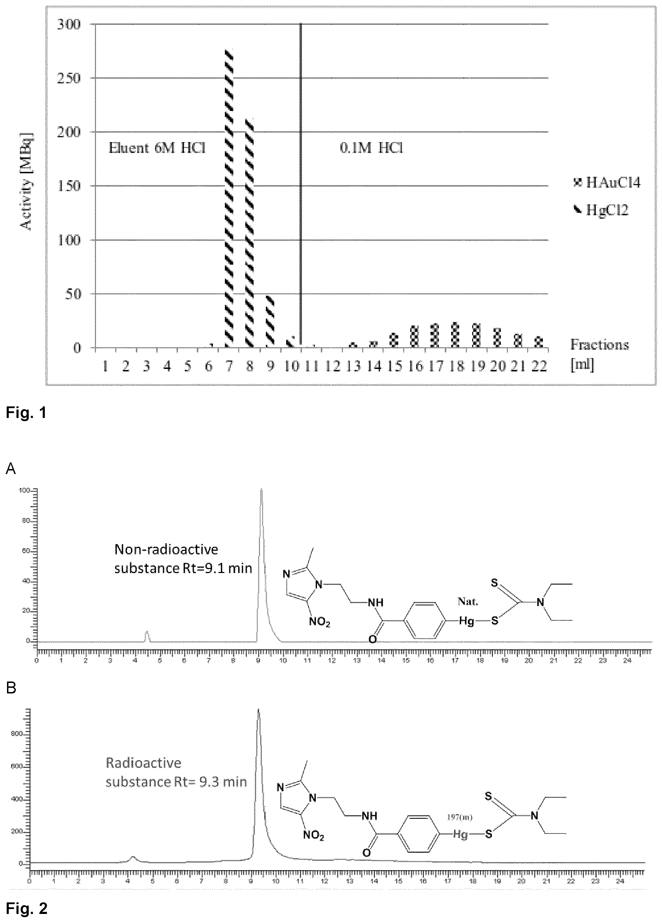

**Fig. 1** shows the fractionated elution of ^{197(m)}Hg mercury chloride in 6 M HCI (two major fractions 7+8 and two minor fractions 9+10) and ¹⁹⁸Au+¹⁹⁶Au containing chloroauric acid in 0.1 M HCI (fractions 13-22).

### 3. Radiolabeling of the organic precursor compound with the no carrier added (NCA) ^{197(m)}Hg by electrophilic substitution

### General synthetic procedure for synthesis of diphenyl^{nat}mercury compounds (reference) - based on Sn-precursors:

A solution of one equivalent mercury (II)-chloride was added to a solution of two equivalents tin-precursor in acetonitrile. The immediately starting precipitation of the product was completed by addition of ice cooled diethyl ether after 2 h mixing at room temperature. Centrifugation followed by washing the residue with cold diethyl ether results in a colorless microcrystalline product.

### Bis(4-(N-succinimidyl)benzoate)mercury (II) (reference)

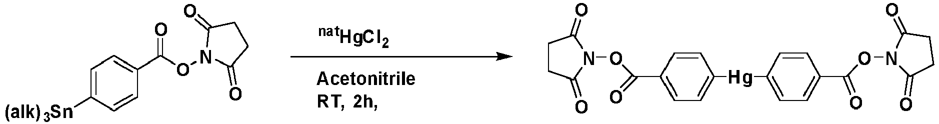

A solution of one equivalent mercury (II)-chloride (5.5 mg, 20 µmol) in 1.5 ml acetonitrile was added to a solution of two equivalents tin-precursor N-succinimidyl-4-(tri-n-butylstannyl)benzoate (21 mg, 41 µmol) in 1.5 ml acetonitrile. The immediately starting precipitation of the product was completed by addition of ice cooled diethyl ether after 2 h mixing at room temperature. Centrifugation followed by washing the residue with cold diethyl ether results in a colorless microcrystalline product.

Chemical Formula: C₂₂H₁₆HgN₂O₈,
Molecular Weight: 636,97 g/mol,
**¹H**-**NMR** (400 MHz, DMSO-D₆) *δ* (ppm): 2.89 (s, 8H); 7.77 (d, 4H); 7.99 (d, 4H),
**¹³C-NMR** (100 MHz, DMSO-D₆) *δ* (ppm): 25.5 (CH₂); 123.5 (C); 128.8 (CH); 137.8 (CH); 161.9 (C); 162.0 (C); 170.3 (C), *yield:* 7 mg (15.4 µmol; 77%),
**ESI⁺** m/z: 637 [M]⁺; 539 [M-NHS]⁺.

### General synthetic procedure for synthesis of radiolabeled diphenyl-mercury species - based on Sn-precursors

The ^{197(m)}Hg chloride stock solution in 0.2 M HCI is adjusted to pH 6 by adding 100 µl 0.2 M 2-(N-morpholino)ethanesulfonic acid (MES) buffer and 5-10 µl 1 M NaOH. A solution of 1-10 µg trialkyltin precursor in 50-100 µl dimethyl sulfoxide (DMSO) is added to this buffered ^{197(m)}Hg chloride solution and mixed at 50°C for 1 h. The completion of the reaction is confirmed by TLC control (acetonitrile (ACN)/H₂O 90:10 (v/v) with 0.1 vol-% trifluoroacetic acid (TFA), instant thin layer chromatography medium (iTLC)-silica gel (SG) and RP18 material).

### [^{197(m)}Hg] Bis(4-(N-succinimidyl)benzoate)mercury(II)

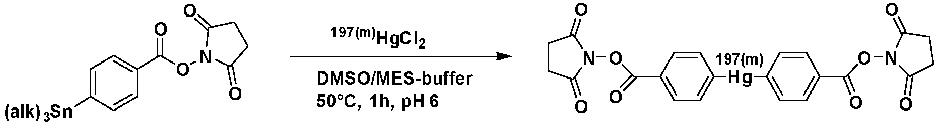

The ^{197(m)}Hg chloride solution in 0.2 M HCI is adjusted to pH 6 by adding 100 µl 0.2 M 2-(N-morpholino)ethanesulfonic acid (MES) buffer and 5-10 µl 1 M NaOH. A solution of 10 µg (20 nmol) N-succinimidyl-4-(tri-n-butylstannyl)benzoate in 100 µl DMSO is added to 110 µl of this buffered ^{197(m)}Hg chloride solution (45 MBq [^{197(m)}Hg] mercury) and mixed at 50°C for 1 h. The completion of the reaction is confirmed by TLC control (ACN/H₂O 90:10 (v/v) with 0.1 vol-% trifluoroacetic acid (TFA), instant thin layer chromatography medium (iTLC)-silica gel (SG) and RP18 material). Radiochemical yield (TLC): ≥ 95 %,
Radiochemical purity (TLC): ≥ 95 %
Radio-TLC: R_{f} = 0.45 (ACN/H₂O 90:10 (v/v) with 1 vol-% trifluoroacetic acid (TFA, RP-18).

### General synthetic procedure for synthesis of diaryl/heteroaryl^{nat}mercury compounds (HPLC reference) - based on B-precursors

### (see ref. Partyka et al., J. Organometallic Chemistry)

A mixture of one equivalent mercury (II)-acetate (5 µmol), ten equivalents boronic acid (50 µmol) and ten equivalents cesium carbonate (50 µmol) in 1 ml propane-2-ol was tempered at 50 °C for 20 h. After cooling and drying the mixture by rotary evaporation the product was extracted from the residue with toluene or THF purified by HPLC and identified by mass spectrometry.

### Di(thiophen-2-yl)mercury

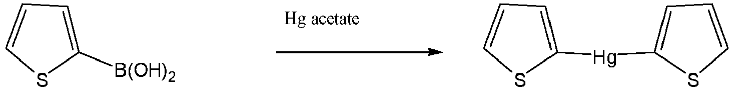

A solution of one equivalent mercury (II)-acetate (1.6 mg, 5 µmol) in 0.5 ml propan-2-ol was added to a solution of ten equivalents 2-thienylboronic acid (6.4 mg, 50 µmol) and cesium carbonate (16 mg, 50 µmol) in 1.0 ml propan-2-ol and mixed at 50 °C for 20 h.
Chemical Formula: C₈H₆HgS₂,
Molecular Weight: 366.85 g/mol,
**ESI⁺ m/z:** 369 [M]⁺.

### Bis(5-carboxythiophen-2-yl)mercury

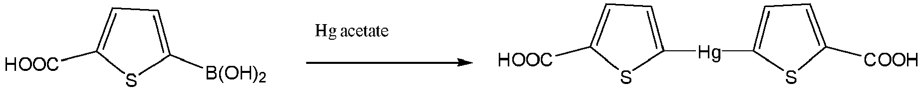

A solution of one equivalent mercury (II)-acetate (1.6 mg, 5 µmol) in 0.5 ml propan-2-ol was added to a solution of ten equivalents 5-(Dihydroxyboryl)-2-thiophenecarboxylic acid (8.5 mg, 50 µmol) and cesium carbonate (16 mg, 50 µmol) in 1.0 ml propan-2-ol and mixed at 50 °C for 20 h.
Chemical Formula: C₁₀H₆HgO₄S₂,
Molecular Weight: 454.86 g/mol,
**ESI⁺ m/z:** 457 [M]⁺.

### Di(ferrocenyl)mercury

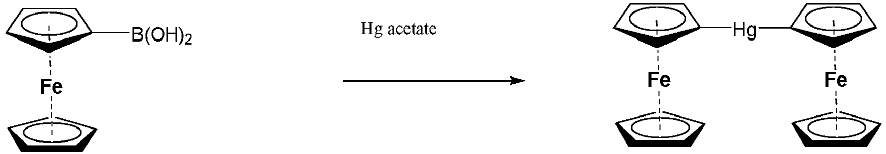

A solution of one equivalent mercury (II)-acetate (1.6 mg, 5 µmol) in 0.5 ml propan-2-ol was added to a solution of ten equivalents ferroceneboronic acid (11.5 mg, 50 µmol) and cesium carbonate (16 mg, 50 µmol) in 1.0 ml propan-2-ol and mixed at 50 °C for 20 h.
Chemical Formula: C₂₀H₁₈Fe₂Hg,
Molecular Weight: 570.64 g/mol,
**ESI⁺ m/z:** 573 [M]⁺.

### Bis(5-carboxypyridin-3-yl)mercury

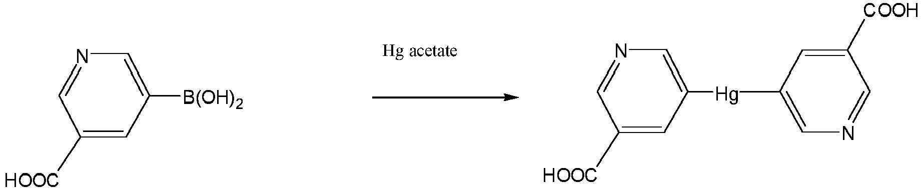

A solution of one equivalent mercury (II)-acetate (1.6 mg, 5 µmol) in 0.5 ml propan-2-ol was added to a solution of ten equivalents 5-(dihydroxyboryl)-3-pyridinecarboxylic acid (8.3 mg, 50 µmol) and cesium carbonate (16 mg, 50 µmol) in 1.0 ml propan-2-ol and mixed at 50 °C for 20 h. Chemical Formula: C₁₂H₈HgN₂O₄,
Molecular Weight: 444.02 g/mol,
**ESI⁺ m/z:** 447 [M]⁺.

### (5-Carboxythiophen-2-yl)(phenyl)mercury

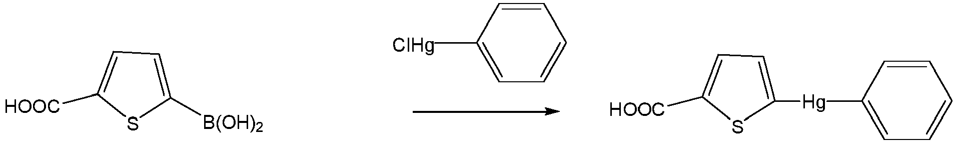

A solution of one equivalent phenylmercury acetate (1.7 mg, 5 µmol)in 0.5 ml propan-2-ol was added to a solution of ten equivalents 5-(dihydroxyboryl)-2-thiophenecarboxylic acid (8.5 mg, 50 µmol) and cesium carbonate (16 mg, 50 µmol) in 1.0 ml propan-2-ol and mixed at 50 °C for 20 h. Chemical Formula: C₁₁H₈HgO₂S,
Molecular Weight: 404.83 g/mol,
**ESI⁺ m/z:** 407 [M]⁺.

### General synthetic procedure for synthesis of radiolabeled diaryl/heteroaryl⁻mercury species

### - based on B-precursors

A solution of 10-100 µg aryl boronic acid precursor in 50-100 µl ethanol is added to the intended amount ^{197(m)}Hg acetate solution in 0.2 M sodium acetate. The pH of the mixture is then adjusted to pH 8 by adding 100 µl 0.2 M 2-[4-(2-hydroxyethyl)piperazin-1-yl]ethanesulfonic acid (HEPES) buffer and shaken at 50°C for 1 h. The completion of the reaction is confirmed by TLC control (acetonitrile (ACN)/H₂O 90:10 (v/v) with 0.1 vol-% trifluoroacetic acid (TFA), instant thin layer chromatography medium (iTLC)-silica gel (SG) and RP18 material).

### Di(thiophen-2-yl)mercury

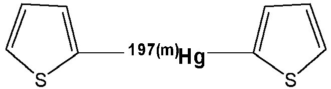

Radiochemical yield (TLC): ≥ 95 %,
Radio-TLC: R_{f} = 0.2 (ACN/H₂O 90:10 (v/v) with 1 vol-% trifluoroacetic acid (TFA, RP-18).

### Bis(5-carboxythiophen-2-yl)mercury

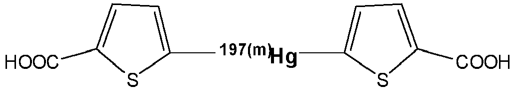

Radiochemical yield (TLC): ≥ 95 %,
Radio-TLC: R_{f} = 0.9 (ACN/H₂O 90:10 (v/v) with 1 vol-% trifluoroacetic acid (TFA, RP-18).

### Di(ferrocenyl)mercury

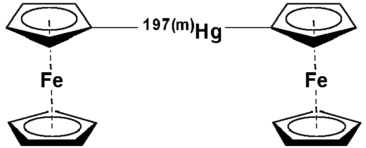

Radiochemical yield (TLC): ≥ 95 %,
Radio-TLC: R_{f} = 0.1 (ACN/H₂O 90:10 (v/v) with 1 vol-% trifluoroacetic acid (TFA, RP-18).

### Bis(5-carboxypyridin-3-yl)mercury

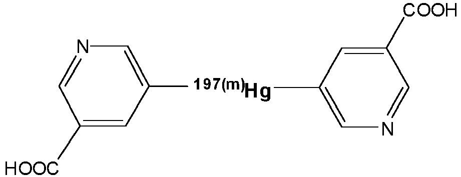

Radiochemical yield (TLC): ≥ 95 %,
Radio-TLC: R_{f} = 0.9 (ACN/H₂O 90:10 (v/v) with 1 vol-% trifluoroacetic acid (TFA, RP-18).

### (5-carboxythiophen-2-yl)(phenyl)mercury

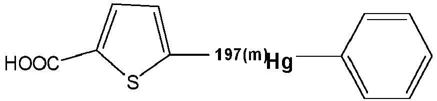

This heteroleptic diaryl mercury compound is accessible in a two-step procedure (analogous to the asymmetric phenylmercury dithiocarbamate derivatives (see next section):

### Step 1: Synthesis of ^{197(m)}Hg phenylmercury chloride

The ^{197(m)}Hg chloride stock solution in 0.2 M HCI is diluted by adding 100 µl water and 100 µl ethanol to improve the solubility of the tin precursor and the lipophilic intermediate. A solution of 10 µg trimethylstannyl benzene precursor in 50 µl dimethyl sulfoxide (DMSO) is added to this acidic ^{197(m)}Hg chloride solution and mixed at 50°C for 1 h. The completion of the reaction is confirmed by TLC control (acetonitrile (ACN)/H₂O 90:10 (v/v) with 0.1 vol-% trifluoroacetic acid (TFA), instant thin layer chromatography medium (iTLC)-silica gel (SG) and RP18 material).

### Step 2: Reaction of the ^{197(m)}Hg phenylmercury chloride with the aryl boronic acid

A solution of 50 µg 5-carboxy-2-thienylboronic acid in 50 µl ethanol is added together with 100 µl 0.2 M sodium acetate to the ^{197(m)}Hg phenylmercury chloride. The pH of the mixture is then adjusted to pH 8 by adding 100 µl 0.2 M 2-[4-(2-hydroxyethyl)piperazin-1-yl]ethanesulfonic acid (HEPES) buffer and shaken at 50°C for 1 h. The completion of the reaction is confirmed by TLC control (acetonitrile (ACN)/H₂O 90:10 (v/v) with 0.1 vol-% trifluoroacetic acid (TFA), instant thin layer chromatography medium (iTLC)-silica gel (SG) and RP18 material).
Radiochemical yield (TLC): ≥ 60 %,
Radio-TLC: R_{f} = 0.45 (ACN/H₂O 90:10 (v/v) with 1 vol-% trifluoroacetic acid (TFA, RP-18).

### Synthesis of asymmetric radiolabeled aryl-mercury-dithiocarbamate derivatives:

### [^{197(m)}Hg] (Diethylcarbamothioyl)thio)(4-((2-(2-methyl-5-nitro-1H-imidazol-1-yl)ethyl)benzoyl-amido)-mercury (II)

### Step 1: Phenyl-^{197(m)}Hg-Cl derivatives

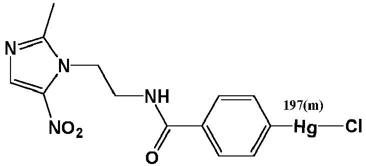

2 µg of the tin precursor *N*-(2-(2-methyl-5-nitro-1H-imidazol-1-yl)ethyl)-4-(tributylstannyl)benzamide (K08-15) dissolved in 20 µl DMSO was added into 50 µl 0.1 M HCI solution containing 45.5 MBq [^{197(m)}Hg]HgCl₂. The reaction mixture was shaken overnight at25 °C (> 12 h). Acidic environment is needed to avoid the formation of symmetric diphenyl mercury species. Excess of organotin precursors were decomposed slowly in acid environment.

### Step 2: Ph-^{197(m)}Hg-dithiocarbamate derivatives

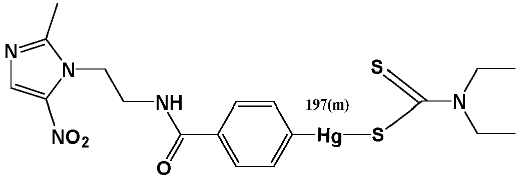

The pH of the phenyl mercury chloride derivatives (step 1) was adjusted to pH 6, adding about 200 µl 0.2 M MES buffer (pH 6.0 to 6.2) and about 10 µl 0.2 M NaOH, before the dithiocarbamate ligand is added. Then 20 µg dithiocarbamate (cw04) containing 50 µl 0.2 M MES buffer (pH 6.0 to 6.2) were added into mixture quickly. Then, the reaction mixture was shaken at 50°C for 60 min.

Radiochemical purity was determined by radio-HPLC (see **Fig. 2**). The non-radioactive reference substance of dithiocarbamate arylmercury derivative was used to confirm the labeling product either. Fig. 2 shows a) Radiochromatogram of Phenyl-^{197(m)}Hg-dithiocarbamate and b) UV-chromatogram of non-radioactive Phenyl-Hg-dithiocarbamate (reference).

### 4. Ester hydrolysis

### Bis(4-carboxyphenyl)mercury(II) (reference)

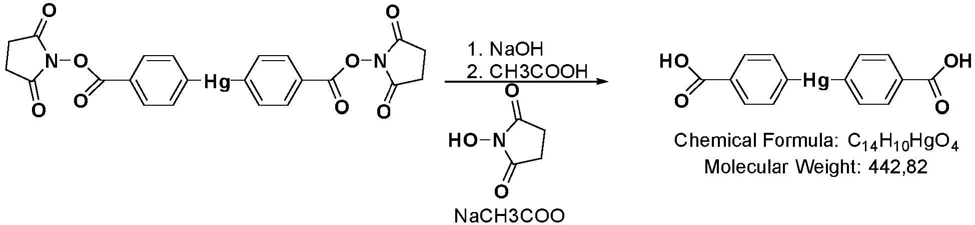

To a solution of 23 mg (36 µmol) Bis(4-(N-succinimidyl)benzoate)mercury(II) in 2 ml dimethylformamide (DMF) 2.88 µl 2.5 N NaOH (72 µmol) and 1 ml water were added. After mixing 2 h at 50°C the completion of the reaction was confirmed by TLC control (DCM/MeOH 50:1 (v/v), DC silica gel 60 F₂₅₄). The pH was adjusted to pH 3 by addition of acetic acid then the solvent was removed by rotary evaporation and residue redissolved in 2 ml DMF. The product was precipitated by addition of 20 ml cold diethyl ether, filtrated and dried under vacuum, resulting in a white solid.
Chemical Formula: C₁₄H₁₀HgO₄,
Molecular Weight: 442,82 g/mol,
**¹H-NMR** (400 MHz, DMSO-D₆, AcOH-D₄) δ (ppm): 7.52 (d, 4H); 7.83 (d, 4H),
**¹³C-NMR** (100 MHz, DMSO-D₆, AcOH-D₄) δ (ppm): 129.6 (CH); 131.0 (C); 137.7 (CH); 161.6 (C); 168.4 (C), yield: 15.3 mg (34 µmol; 94%),
**ESI⁺ m/z:** 443 [Hg-M]⁺.

### [^{197(m)}Hg] Bis(4-carboxyphenyl)mercury (II)

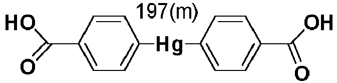

The solution of [^{197(m)}Hg] Bis(4-(N-succinimidyl)benzoate)mercury (II) is adjusted to pH 9 by adding 10 µl 1 M NaOH and mixed for 1 h at 50°C. The completion of the reaction is confirmed by TLC control (ACN/H₂O 90:10 (v/v) with 0.1 vol-% TFA, ITLC-SG and RP18 material). Finally, the pH is adjusted to pH 6-7 by addition of 10 µl 1 M HCl.
Radiochemical yield (TLC): ≥ 95 %,
Radiochemical purity (TLC): ≥ 95 %
Radio-TLC: R_{f}= 0.6 (ACN/H₂O 90:10 (v/v) with 0.1 vol-% TFA, RP-18).

### 5. Synthesis of the [^{197(m)}Hg] Bis(4-carboxyphenyl)mercury (II)-mAb Cetuximab (C225) conjugate by prelabeling with the labeled active ester

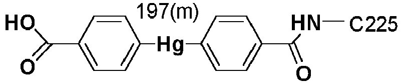

The solution of [^{197(m)}Hg] Bis(4-(N-succinimidyl)benzoate)mercury (II) is added to a solution of 1 mg size-exclusion chromatography (SEC) purified C225 antibody in HEPES buffer at pH 8. After mixing the pH is adjusted to pH 8.5. After 1 h at 37°C the progress of the reaction is confirmed by TLC control. (ACN/H₂O 90:10 (v/v) with 0.1 vol-% TFA, ITLC-SG and RP18 material). Unreacted active ester residues were quenched by adding 10 µl 1 M tris(hydroxymethyl)aminomethane (TRIS) solution and separated using a PD10 desalting column.
Radiochemical yield (TLC): ≥ 50-70 %,
Radiochemical purity (TLC): ≥ 95 %,
Radio-TLC: R_{f}= 0 (ACN/H₂O 90:10 (v/v) with 0.1 vol-% TFA, RP-18).

### Cited non-patent literature

R.L. Greif, W.J. Sullivan, G.S. Jacobs, R.F Pitts (1956) Distribution of radiomercury administered as labelled chlormerodrin (neohydrin) in the kidneys of rats and dogs. J. Clin. Investig. 35, 38-43. D. B. Sodee (1964) Letters to the Editor. Hg-197 as a Scanning nuclide. J. Nuc. I. Med. 5, 1964, 74-75.
B. Matricali (1969) Brain scanning by means of 197Hg-labelled neohydrin. Psychiatr. Neurol. Neurochir. 72, 89-95.
M. Walther, S. Preusche, S. Bartel, G. Wunderlich, R. Freudenberg, J. Steinbach, H.-J. Pietzsch (2015) Theranostic mercury: 197(m)Hg with high specific activity for imaging and therapy. Applied Radiation and Isotopes 97, 177-181.
M. Walther, O. Lebeda, S. Preusche, H.-J. Pietzsch, J. Steinbach (2016) Theranostic mercury Part 1: A New Hg/Au separation by a resin based method. Abstract 16th International Workshop on Targetry and Target Chemistry.
G. N. George, R. C. Prince, J. Gailer, G. A. Buttigieg, M. B. Denton, H. H. Harris, I. J. Pickering (2004) Mercury Binding to the Chelation Therapy Agents DMSA and DMPS and the Rational Design of Custom Chelators for Mercury. Chem. Res. Toxicol. 17, 999-1006.
F. S. Mishkin (1966) Clinical brain scanning with 203Hg Neohydrin. J. Indiana State Med. Assoc. 59 (12), 1435-1438.
T. W. Clarkson, L. Magos (2006) The Toxicology of Mercury and Its Chemical Compounds. Critical Reviews in Toxicology. 36, 609-662.
Remington's Pharmaceutical Sciences (1975) 15th Edition. Editor: A. Osol and J. E. Hoover. Mack Publishing Co., Easton, PA 18042.
D.V. Partyka, T.G. Gray (2009) Facile syntheses of homoleptic diarylmercurials via arylboronic acids. J. Organometallic Chem. 694, 213-218.

## Claims

1. A ^{197(m)}Hg compound according to one of the following formulas (I), (Ia), (lb), (Ic), (Ia_{bridge}), (Ib_{bridge}), (Ic_{bridge}) or (VI) 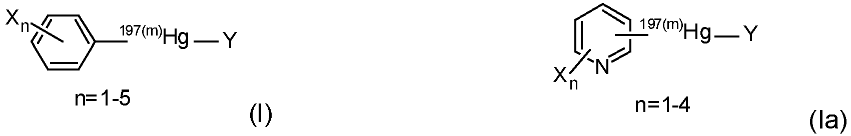 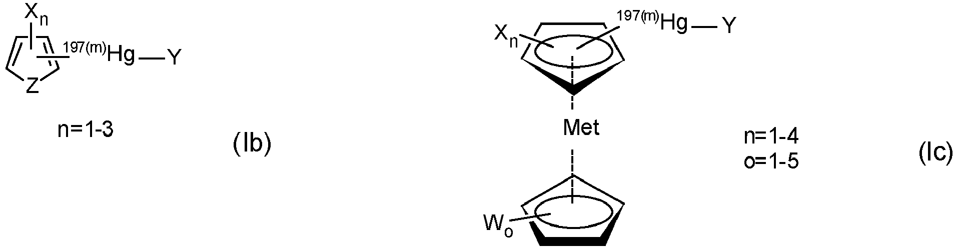 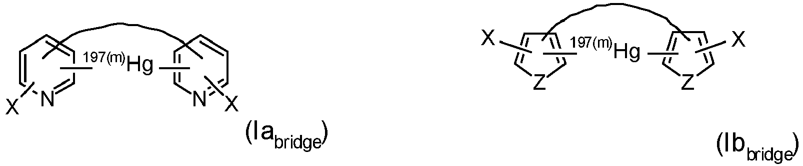 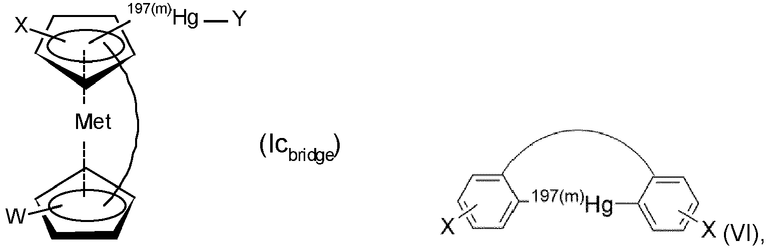
wherein each X and each W are independently selected from H, unsubstituted or substituted alkyl groups, alkoxy groups with formula -OR¹, amide groups with formula -CON(R¹)₂, carboxy groups with formula -COOR¹, aryl and heteroaryl groups,
wherein R¹ is selected from H, unsubstituted or substituted C1 to C15-alkyl, succinimidyl, -aryl or -heteroaryl groups,
wherein Y is selected from substituted dithiocarbamates, substituted thiolates, unsubstituted or substituted phenyl and other aryl or heteroaryl group,
wherein Z is selected from CH, S, N, and O,
wherein Met is selected from Fe, Cr, Mn, Mo, Ru, and Rh.

2. ^{197(m)}Hg compound according to claim 1, wherein n and o are 1.,

3. ^{197(m)}Hg compound according to claim 1 or 2 having a specific activity of at least 100 GBq/µmol based on the amount of mercury.

4. ^{197(m)}Hg compound according to one of the claims 1 to 3, wherein Xₙ and/or Y comprise at least one amino acid, peptide, protein, antibody, oligonucleotide, alkaloid residue and/or aliphatic spacer.

5. ^{197(m)}Hg compound according to one of the claims 1 to 4, wherein -Y is selected from unsubstituted or substituted phenyl groups as shown in formula (IV) 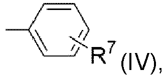
wherein R⁷ is selected from H, unsubstituted or substituted alkyl groups, alkoxy groups with formula -OR⁸, amide groups with formula -CON(R⁸)₂, carboxy groups with formula -COOR⁸, aryl or heteroaryl groups,
wherein R⁸ is selected from H, unsubstituted or substituted C1 to C15-alkyl, succinimidyl, -aryl or -heteroaryl groups.

6. ^{197(m)}Hg compound according to one of the claims 1 to 5 for use in nuclear medical diagnostics, endoradionuclide therapy or for use in the treatment of cancer.

7. A method for the production of ^{197(m)}Hg compounds according to one of the claims 1 to 5 comprising the steps:
a) Provision of an organic precursor compound,
b) Synthesis of no carrier added (NCA) ^{197(m)}Hg,
c) Radiolabeling of the organic precursor compound with the no carrier added (NCA) ^{197(m)}Hg by electrophilic substitution.

8. The method according claim 7, wherein the organic precursor compound is an organotin precursor compound, a boron precursor compound or a silicon precursor compound.

9. The method according to claim 7 or 8, wherein the organic precursor compound is a trialkyltin precursor compound.

10. The method of one of the claims 7 to 9, wherein the synthesis of NCA ^{197(m)}Hg according to step b) is carried out by irradiation of gold (Au) with a cyclotron.

11. The method according to one of the claims 7 to 10, wherein the radiolabeling of the organic precursor compound according to step c) is carried out at a pH value between pH 1.0 and 5.0 to form asymmetric ^{197(m)}Hg compounds.

12. The method according to one of the claims 7 to 11, wherein the radiolabeling of the organic precursor compound according to step c) is followed by reaction of activated ester groups by ester hydrolysis, reaction with amino groups or reaction with hydroxyl groups of an amino acid, peptide, protein, antibody, oligonucleotide, alkaloid residue and/or aliphatic spacer.

13. Use of an organic precursor compound according to formulas (I_{prec}), (Ia_{prec}), (Ib_{prec}) or (Ic_{prec}) 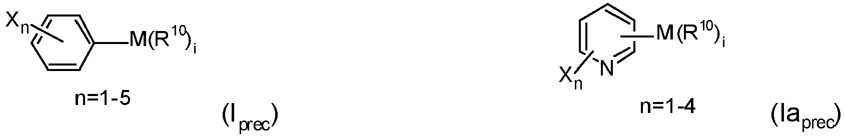 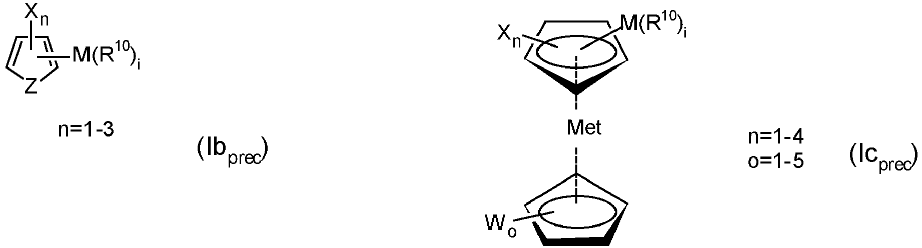
wherein each X and each W are independently selected from H, unsubstituted or substituted alkyl groups, alkoxy groups with formula -OR¹, amide groups with formula - CON(R¹)₂, carboxy groups with formula -COOR¹, aryl or heteroaryl groups,
wherein R¹ is selected from H, unsubstituted or substituted C1 to C15-alkyl, -aryl or -heteroaryl groups,
Z is selected from CH, S, N, and O,
M is Sn, B or Si;
wherein Met is selected from Fe, Cr, Mn, Mo, Ru and Rh,
R¹⁰ is selected from H, unsubstituted or substituted C1 to C15-alkyl, -aryl or -heteroaryl groups, and
i is 2 or 3,
in the production of the ^{197(m)}Hg compounds according to one of the claims 1 to 5.

## Patentansprüche

1. ^{197(m)}Hg-Verbindung gemäß einer der folgenden Formeln (I), (Ia), (Ib), (Ic), (Ia_{Brücke}), (Ib_{Brücke}), (Ic_{Brücke}) oder (VI) 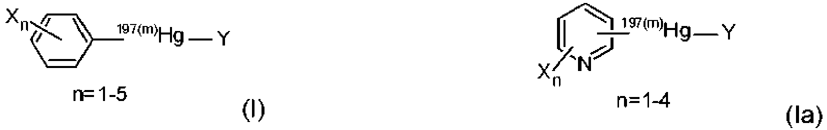 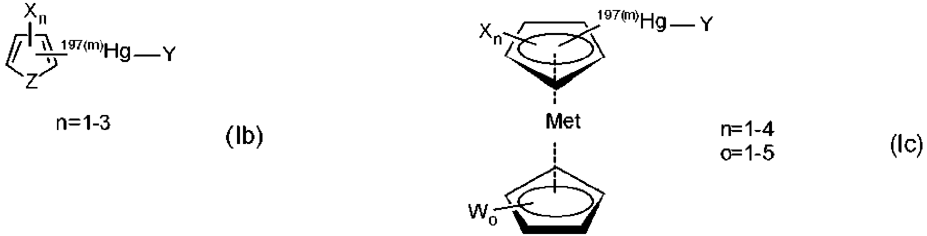 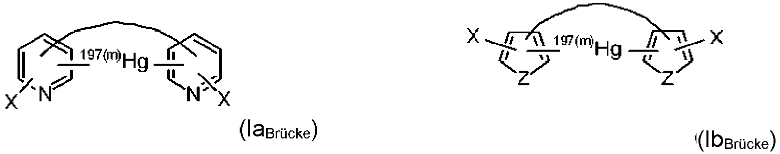 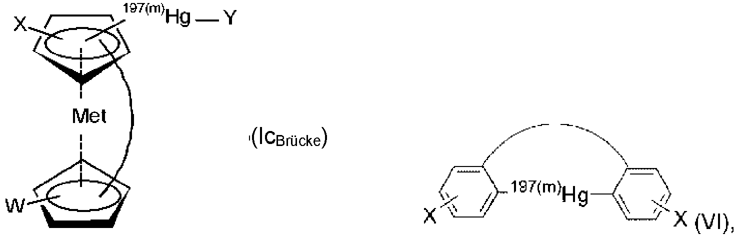
wobei jedes X und jedes W unabhängig aus H, unsubstituierten oder substituierten Alkylgruppen, Alkoxygruppen mit der Formel -OR¹, Amidgruppen mit der Formel - CON(R¹)₂, Carboxygruppen mit der Formel -COOR¹, Aryl- und Heteroarylgruppen ausgewählt sind,
wobei R¹ aus H, unsubstituierten oder substituierten C1- bis C15-Alkyl-, Succinimidyl-, -Aryl- oder -Heteroarylgruppen ausgewählt ist,
wobei Y aus substituierten Dithiocarbamaten, substituierten Thiolaten, unsubstituiertem oder substituiertem Phenyl und einer anderen Aryl- oder Heteroarylgruppe ausgewählt ist,
wobei Z aus CH, S, N und O ausgewählt ist,
wobei Met aus Fe, Cr, Mn, Mo, Ru und Rh ausgewählt ist.

2. ^{197(m)}Hg-Verbindung nach Anspruch 1, wobei n und o 1 sind.

3. ^{197(m)}Hg-Verbindung nach Anspruch 1 oder 2, die eine spezifische Aktivität von wenigstens 100 GBq/µmol basierend auf der Quecksilbermenge aufweist.

4. ^{197(m)}Hg-Verbindung nach einem der Ansprüche 1 bis 3, wobei Xₙ und/oder Y wenigstens eine Aminosäure, wenigstens ein Peptid, wenigstens ein Protein, wenigstens einen Antikörper, wenigstens ein Oligonukleotid, wenigstens einen Alkaloidrest und/oder wenigstens einen aliphatischen Spacer umfasst.

5. ^{197(m)}Hg-Verbindung nach einem der Ansprüche 1 bis 4, wobei -Y aus unsubstituierten oder substituierten Phenylgruppen, wie in Formel (IV) gezeigt, ausgewählt ist 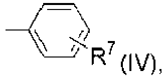
wobei R⁷ aus H, unsubstituierten oder substituierten Alkylgruppen, Alkoxygruppen mit der Formel -OR⁸, Amidgruppen mit der Formel -CON(R⁸)₂, Carboxygruppen mit der Formel -COOR⁸, Aryl- oder Heteroarylgruppen ausgewählt ist,
wobei R⁸ aus H, unsubstituierten oder substituierten C1- bis C15-Alkyl-, Succinimidyl-, -Aryl- oder -Heteroarylgruppen ausgewählt ist.

6. ^{197(m)}Hg-Verbindung nach einem der Ansprüche 1 bis 5 zur Verwendung in nuklearmedizinischer Diagnostik, Endoradionuklidtherapie oder zur Verwendung bei der Behandlung von Krebs.

7. Verfahren für die Herstellung von ^{197(m)}Hg-Verbindungen nach einem der Ansprüche 1 bis 5, das die folgenden Schritte umfasst:
a) Bereitstellung einer organischen Vorläuferverbindung,
b) Synthese von ^{197(m)}Hg ohne Trägerzusatz (no carrier added - NCA),
c) radioaktives Markieren der organischen Vorläuferverbindung mit dem ^{197(m)}Hg ohne Trägerzusatz (NCA) durch elektrophile Substitution.

8. Verfahren nach Anspruch 7, wobei die organische Vorläuferverbindung eine Organozinnvorläuferverbindung, eine Borvorläuferverbindung oder eine Siliziumvorläuferverbindung ist.

9. Verfahren nach Anspruch 7 oder 8, wobei die organische Vorläuferverbindung eine Trialkyl-Zinn-Vorläuferverbindung ist.

10. Verfahren nach einem der Ansprüche 7 bis 9, wobei die Synthese von NCA ^{197(m)}Hg gemäß Schritt b) durch Bestrahlung von Gold (Au) mit einem Zyklotron durchgeführt wird.

11. Verfahren nach einem der Ansprüche 7 bis 10, wobei das radioaktive Markieren der organischen Vorläuferverbindung gemäß Schritt c) bei einem pH-Wert zwischen pH 1,0 und 5,0 durchgeführt wird, um asymmetrische ^{197(m)}Hg-Verbindungen auszubilden.

12. Verfahren nach einem der Ansprüche 7 bis 11, wobei auf das radioaktive Markieren der organischen Vorläuferverbindung gemäß Schritt c) eine Reaktion von aktivierten Estergruppen durch Esterhydrolyse, die Reaktion mit Aminogruppen oder die Reaktion mit Hydroxylgruppen von Aminosäure, Peptid, Protein, einem Antikörper, Oligonukleotid, einem Alkaloidrest und/oder einem aliphatischen Spacer folgt.

13. Verwendung einer organischen Vorläuferverbindung gemäß den Formeln (I_{Vorl}), (Ia_{Vorl}), (Ib_{Vorl}) oder (Ic_{Vorl}) 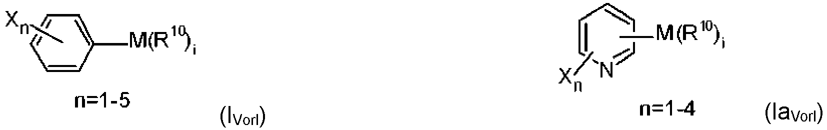 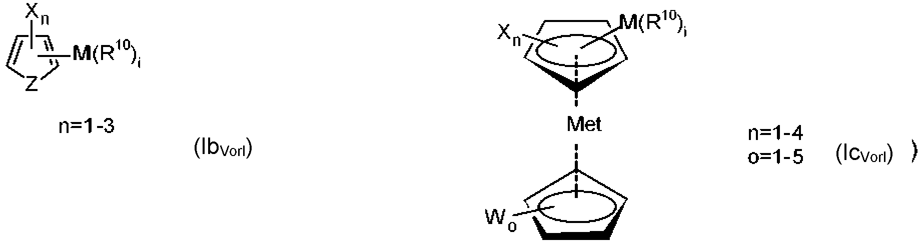
wobei jedes X und jedes W unabhängig aus H, unsubstituierten oder substituierten Alkylgruppen, Alkoxygruppen mit der Formel -OR¹, Amidgruppen mit der Formel - CON(R¹)₂, Carboxygruppen mit der Formel -COOR¹, Aryl- oder Heteroarylgruppen ausgewählt sind,
wobei R¹ aus H, unsubstituierten oder substituierten C1- bis C15-Alkyl-, -Aryl- oder-Heteroarylgruppen ausgewählt ist,
Z aus CH, S, N und O ausgewählt ist,
M Sn, B oder Si ist;
wobei Met aus Fe, Cr, Mn, Mo, Ru und Rh ausgewählt ist,
R¹⁰ aus H, unsubstituierten oder substituierten C1- bis C15-Alkyl-, -Aryl- oder - Heteroarylgruppen ausgewählt ist und
i 2 oder 3 ist,
bei der Herstellung der ^{197(m)}Hg-Verbindungen nach einem der Ansprüche 1 bis 5.

## Revendications

1. Composé ^{197(m)}Hg selon l'une des formules suivantes (I), (Ia), (Ib), (Ic), (Iaₚₒₙₜ), (Ibₚₒₙₜ), (Icₚₒₙₜ) ou (VI) 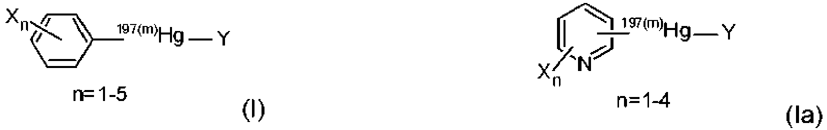 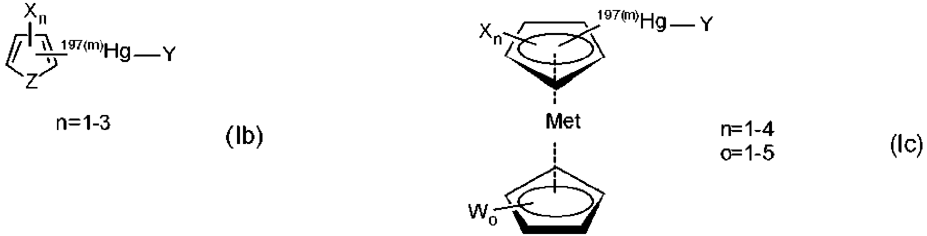 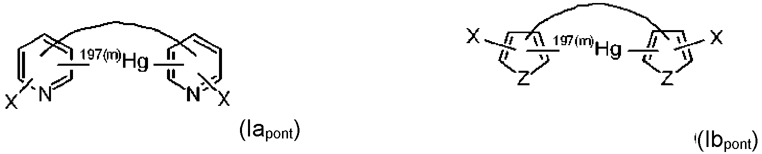 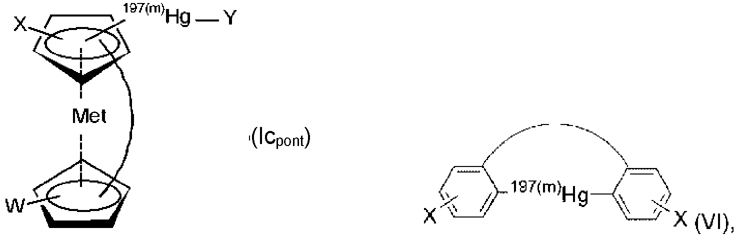
chaque X et chaque W étant sélectionnés indépendamment parmi H, des groupes alkyle non substitués ou substitués, des groupes alcoxy de formule -OR¹, des groupes amide de formule - CON(R¹)₂, des groupes carboxy de formule -COOR¹, des groupes aryle et hétéroaryle,
R¹ étant sélectionné parmi H, des groupes -alkyle, succinimidyle, -aryle ou -hétéroaryle en C1 à C15 non substitués ou substitués,
Y étant sélectionné parmi les dithiocarbamates substitués, les thiolates substitués, le phényle non substitué ou substitué et un autre groupe aryle ou hétéroaryle,
Z étant sélectionné parmi CH, S, N et O,
Met étant sélectionné parmi Fe, Cr, Mn, Mo, Ru et Rh.

2. Composé ^{197(m)}Hg selon la revendication 1, dans lequel n et o représentent 1.

3. Composé ^{197(m)}Hg selon la revendication 1 ou 2 ayant une activité spécifique d'au moins 100 GBq/µmol sur la base de la quantité de mercure.

4. Composé ^{197(m)}Hg selon l'une des revendications 1 à 3, dans lequel Xₙ et/ou Y comprennent au moins un acide aminé, peptide, protéine, anticorps, oligonucléotide, résidu alcaloïde et/ou espaceur aliphatique.

5. Composé ^{197(m)}Hg selon l'une des revendications 1 à 4, dans lequel -Y est sélectionné parmi des groupes phényle non substitués ou substitués tels que représentés dans la formule (IV) 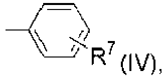
R⁷ étant sélectionné parmi H, des groupes alkyle non substitués ou substitués, des groupes alcoxy de formule -OR⁸, des groupes amide de formule -CON(R⁸)₂, des groupes carboxy de formule -COOR⁸, des groupes aryle ou hétéroaryle,
R⁸ étant sélectionné parmi H, des groupes -alkyle, succinimidyle, -aryle ou -hétéroaryle en C1 à C15 non substitués ou substitués.

6. Composé ^{197(m)}Hg selon l'une des revendications 1 à 5 destiné à être utilisé pour des diagnostics médicaux nucléaires, une thérapie utilisant des endoradionucléides ou destiné à être utilisé pour le traitement d'un cancer.

7. Procédé pour la production de composés ^{197(m)}Hg selon l'une des revendications 1 à 5 comprenant les étapes consistant à :
a) Fournir un composé précurseur organique,
b) Synthétiser du ^{197(m)}Hg sans support ajouté (NCA),
c) Marquer radioactivement le composé précurseur organique avec le ^{197(m)}Hg sans support ajouté (NCA) par substitution électrophile.

8. Procédé selon la revendication 7, dans lequel le composé précurseur organique est un composé précurseur d'organo-étain, un composé précurseur de bore ou un composé précurseur de silicium.

9. Procédé selon la revendication 7 ou 8, dans lequel le composé précurseur organique est un composé précurseur de trialkyl-étain.

10. Procédé selon l'une des revendications 7 à 9, dans lequel la synthèse de ^{197(m)}Hg NCA selon l'étape b) est réalisée par irradiation d'or (Au) avec un cyclotron.

11. Procédé selon l'une des revendications 7 à 10, dans lequel le marquage radioactif du composé précurseur organique selon l'étape c) est réalisé à une valeur de pH comprise entre pH 1,0 et 5,0 pour former des composés de ^{197(m)}Hg asymétriques.

12. Procédé selon l'une des revendications 7 à 11, dans lequel le marquage radioactif du composé précurseur organique selon l'étape c) est suivi d'une réaction de groupes ester activés par hydrolyse d'ester, réaction avec des groupes amino ou réaction avec des groupes hydroxyle d'un acide aminé, peptide, protéine, anticorps, oligonucléotide, résidu alcaloïde et/ou espaceur aliphatique.

13. Utilisation d'un composé précurseur organique selon les formules (I_{prec}), (Ia_{prec}), (Ib_{prec}) ou (Ic_{prec}) 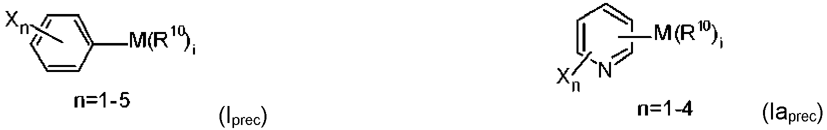 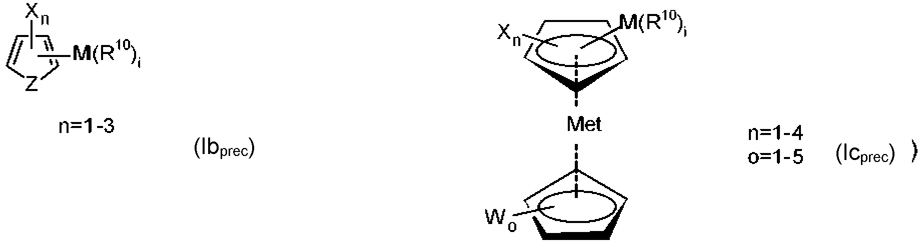
chaque X et chaque W étant indépendamment sélectionnés parmi H, des groupes alkyle non substitués ou substitués, des groupes alcoxy de formule -OR¹, des groupes amide de formule - CON(R¹)₂, des groupes carboxy de formule -COOR¹, des groupes aryle ou hétéroaryle,
R¹ étant sélectionné parmi H, des groupes -alkyle, -aryle ou -hétéroaryle en C1 à C15 non substitués ou substitués,
Z étant sélectionné parmi CH, S, N et O,
M représentant Sn, B ou Si ;
Met étant sélectionné parmi Fe, Cr, Mn, Mo, Ru et Rh,
R¹⁰ étant sélectionné parmi H, des groupes -alkyle, -aryle ou -hétéroaryle en C1 à C15 non substitués ou substitués, et
i représentant 2 ou 3,
dans la production des composés ^{197(m)}Hg selon l'une des revendications 1 à 5.
